# EUROPEAN PATENT APPLICATION

(11) **EP 4 796 142 A2**
(43) Date of publication of application: **26.08.2026**
(21) Application number: 26187203.0
(22) Date of filing: 15.11.2021
(51) Int. Cl.: A61M 5/14

(54) **A BREATHING SYSTEM FOR A PATIENT TO BREATHE THROUGH AND INHALE A SUBSTANCE FROM**

(30) Priority: 14.11.2020 GB 202017957
(62) Document number(s) of the earlier application(s) in accordance with Art. 76 EPC: 21830387.3 / 4 243 906
(71) Applicant: INSPIRED VENTILATION LTD, London W1G 6NE (GB)
(72) Inventor: DARWOOD, Alastair Rupert Joseph, London, W1G 6NE (GB)
(74) Representative: Snipe, Benjamin Thomas Fletcher

(57) **Abstract**

A breathing system is provided for a patient to inhale an analgesic, anaesthetic and/or sedative substance from repeatedly and consecutively. The breathing system comprises: a mask (10) mountable on the patient, so that inhaling and exhaling occur through the mask; adding means (14; 30, 33) for adding the substance to a gas to be inhaled, wherein the substance is a volatile liquid, comprising vaporisation means for vaporising of the substance into the gas, wherein the vaporisation means is mounted in or on the mask so as to be fixedly disposed relative thereto and mountable on a face of the patient, a user control (18); a control means (16), the user control being coupled to the control means to provide input thereto in response to operation of the user control by the patient, and wherein the control means is configured to control the adding by the adding means using the vaporisation means dependent at least on the input.

## Description

### Field of the Invention

The invention relates to breathing system for mounting on a patient so that the patient must breathe through the breathing system, and for adding a substance to gas to be inhaled through the breathing system, particularly where the substance has sedative, anaesthetic and/or analgesic effect on the patient.

### Background

Intravenous administration of analgesic agents, such as opiates, is known where administration is partially controlled by the patient. An administration system may include a syringe driver to provide a background infusion of a drug intravenously at a continuous background rate. A button provided to a patient may be pressed by the patient to provide a drug bolus to address worsening pain. Following administration of this bolus, the administration system may return to providing the drug at the background rate. The administration system may also include software-implemented controls that reduce or prevent risk of overdose by preventing multiple boluses being administered over a predetermined time period. Such an administration system enables a patient to exert some control over administration of the drug while risk of overdose is managed.

Inhalation analgesia advantageously avoids need for intravenous access. For example, an Schimmelbusch mask is an open breathing system for delivering an anaesthetic. The device consists of a wire frame which is covered with several beds of gauze and applied to the patient's face over the mouth and nose. Then high-volatility anaesthetic, such as diethyl ether or halothane and historically chloroform, is dripped on it, allowing the patient to inhale a mix of the evaporated anaesthetic and air.

In some breathing systems, patients may control administration of nitrous oxide gas. In this case a patient may inhale gas through a hand-held regulator when in pain, titrating to good effect. If too much is consumed, analgesic and hypnotic effects prevent continued consumption. If these effects occur, then when reduced, the patient may recommence inhalation of further nitrous oxide gas. Nitrous oxide gas is provided in compressed form in a canister and is cumbersome to transport and use. Alternatives such as methoxyflurane inhalers may be used in a similar manner, as described in a known patent document US3565071A. This document describes placing of a volatile anaesthetic agent into a vaporisation unit including a fabric wick. The wick resides in a cylindrical tube with a mouthpiece and a one-way valve. In use, a patient grasps the tube and inhales through the mouthpiece drawing air into their lungs through the vaporisation unit, thereby self-administering the agent. Simpler approaches to patient-controlled analgesia are also known, where a patient may self-administer a lollipop containing opiate buccally.

A number of drawbacks occur with the above described approaches to self-administration of analgesics. In a stressful or traumatic scenario, it is often challenging to convey to a patient how to self-administer. This results in poor use of an administration system and may reduce analgesic effect. In addition, the above described approaches may require a degree of manual dexterity to hold a control device or a lollipop, for example. This may not be possible where trauma is significant or where a patient has reduced consciousness. Continuous drug administration or intramuscular depot injections are suboptimal due to administration complexity and overdose risks.

It is an object of the present invention to improve upon the above-mentioned approaches.

### Summary of the Invention

According to the present invention, there is provided a breathing system for a patient to inhale a substance from, comprising: a patient airway interface mountable on the patient so that inhaling and exhaling occur through the patient airway interface; adding means for adding the substance to gas to be inhaled, wherein the substance is provided in liquid or powder form, and vaporisation of the substance into the gas is facilitated; a user control; and a control means, the user control being coupled to the control means to provide inputs, for example trigger signals, thereto in response to operation of the user control.

The breathing system overcomes the above described drawbacks.

The control means may be configured to store information indicative of a time of received inputs and/or of amounts of the substance to be added, and to control the adding means dependent at least on: a most recent input received from the user control, and stored information indicative of times of previously received inputs and/or of amounts of the substance added further to the previously received inputs.

The control means may be configured to: determine dependent on said stored information and the most recently received input to increase or decrease the amount of the substance added to the gas; and control the adding unit accordingly.

The control means may be configured to increase the amount of the substance added to the gas if a frequency of the inputs over a predetermined first time period is greater than a first threshold, and/or to decrease the amount of the substance added to the gas if the frequency of the inputs over a predetermined second time period is less than a second threshold.

The control means is coupled to an alerting means, wherein the control means is configured to determine an alert condition, and to cause communication of the alert condition to the patient and/or an operator dependent on at least one rule being met.

The at least one rule may require that an input from the user control is not received within a predetermined period. The at least one rule may require that a rate of inputs received at the control means exceeding a threshold rate. The at least one rule may require that the control means determines that the substance is to be added at a rate exceeding a predetermined maximum rate. The at least one rule may require that a predetermined maximum amount of the substance has been added to the gas. Finally, the at least one rule may require that inputs from the user control is an abnormal pattern of input, the abnormal pattern being determined using a statistical technique. The statistical technique may include determination of a baseline, comparison to the baseline, and the determining of an abnormal pattern may depend on a result of the comparison.

The adding means may comprise a vaporisation means for vaporising the substance.

The breathing system may include a first valve located and configured to prevent inhaled gas from passing through the vaporisation means and to permit gas from the vaporisation means with the substance in from passing through it. The breathing system may include a second valve located and configured to permit exhaled gas from passing through it to the environment and to prevent environmental air from entering the breathing system.

The vaporisation means may be arranged to spread the substance, to increase surface area thereof, thereby to facilitate evaporation of the substance.

The vaporisation means may comprise a material located for gas to flow over or through it, and from which the substance can evaporate. The vaporisation means may be configured to disperse the substance on and/or through the material. Alternatively, to increase or decrease the amount of material exposed to gas flow, for example, using a retractable cover.

The material may be such as to wick the substance from a reservoir of the substance, thereby to disperse the substance through the material.

The vaporisation means may further comprise a spraying device for spraying the substance onto the material.

The adding means may comprise a pump means for pumping the substance onto the material. For example, the pump may be a peristaltic pump, a piezoelectric pump, or pump with a venturi nozzle. The pump may be coupled to a reservoir of the substance.

The pump means may comprise a plurality of tubes opening at a plurality of spaced locations on or through the material. The pump means may be configured to pump the substance through the tubes to deposit the substance at said spaced locations.

The vaporisation means may comprise a heater for heating the substance to cause or facilitate the vaporisation.

The breathing system may further comprise an airway coupled to the patient airway interface including an opening for intake of gas (e.g. air), wherein inhalation causes intake of said gas into the airway, wherein the adding means is arranged to add the substance to the gas in the airway.

The adding means may further comprise a flow control configured to prevent or permit flow of the gas with the substance in into the patient airway interface from the vaporisation means, and to permit or prevent flow of gas without the substance into the patient airway interface. The control means may be coupled to the flow control and configured to control the flow control.

The flow control may be configured with at least one gate. The at least one gate may have a first configuration in which flow from the vaporisation chamber is prevented and flow from the airway is permitted, and a second configuration in which flow from the vaporisation chamber is permitted and flow from the airway is prevented. The flow control may be controllable to move the flow control between the first and second configurations.

The at least one gate may be a single gate hingedly mounted and moveable by the flow control between a first position in which the flow control is in the first configuration and a second position in which the flow control is in the second configuration.

The at least one gate may comprise a first gate operable to control flow of gas from the vaporisation chamber and a second gate operable to control flow of gas from the airway.

An intake of the vaporisation means may be coupled to the airway, such that gas in the vaporisation chamber is drawn from the airway.

The adding means may further comprise means for biasing towards the first configuration.

The flow control may include at least one actuator controllable to move the at least one gate. The control means may be coupled to the at least one actuator to control movement of the at least one gate.

The flow control and/or the control means may be configured to increase or decrease gas flow via the vaporisation means in inverse relation to increase or decrease of flow from the airway.

The patient airway interface may be provided separately to at least some of the rest of the breathing system. The patient airway interface may include a first connector and the rest of the breathing system may include a second connector connectable to the first connector to enable gas flow from the connector into the patient airway interface.

The substance may be for analgesic, anaesthetic and/or sedative effect on the patient. For example, the substance may be methoxyflurane, adrenaline, salbutamol or ketamine.

The adding means and/or one or more parts (e.g. the vaporisation means or parts of the vaporation means such as the material, pumping means and/or a substance reservoir) thereof and/or the control means may be mounted on or in the patient airway interface so as to be fixedly disposed relative thereto. When the breathing system is in use, they may thus be mounted on the face of the patient. In an example, at least some parts of the adding means (e.g. the material) and optionally the control means are included in a patient airway interface in the form of a mask. The patient airway interface may be able to carry the weight of the adding means and the control means.

The material may be planar. An inhalation path may pass perpendicularly with respect to the plane of the material. The material may be mounted proximal a face of the patient. The breathing system may include a housing for retaining the material.

The vaporisation means may include a substance intake port, which may be formed with the housing. The pumping means may be coupled to the substance intake port to push substance to the material through the substance intake port.

In this case, the pumping means may be located physically spaced from the patient airway interface and coupled to the substance intake port with a tube. For example, the pumping means may be co-located and physically coupled to the user control. The control means may be located physically spaced from the patient airway interface and coupled to the substance intake port with a tube.

Where the adding means and/or the one or more parts thereof is mounted on or in the patient airway interface, the control means may be partially located in the user control and partially located on or in the patient airway interface. Thus, the control means may be configured to receive mechanical input (for example pneumatic input) from the user control and control the adding by the adding means in response thereto. In this case, the housing may include an input port for receiving the mechanical input.

The control means may be configured to control the adding unit to add the substance to gas at a background rate in absence of received input.

The user control may be fixable to the patient so that the patient can operate the user control in a user action, thereby to send information indicative of substance being wanted to the control means.

The breathing system may further comprise at least one sensor device for detecting a condition of the patient and providing sensor information indicative of the condition to the control means, wherein the control means is configured to control the adding means dependent also on the sensor information.

The condition may be one of: heart rate, blood pressure; oxygen saturation level; respiratory rate; and patient consciousness level.

The control means may include a processing means and a memory means having computer program code stored thereon, wherein the processing means, with the memory means and the code, provides the functionality set out above. The information indicative of times and/or amounts may be stored in the memory means.

The control means may comprise means (e.g. a bellows) that can be expanded to cause the adding of the substance. In this case, the user control is operable to cause increase of pressure within the expandable means, thereby to cause the expansion. The biasing means biases the expandable means towards a contracted state. The control means may also include a valve configured to permit exit of air from within the expandable means enabling the biasing means to return the expandable means to its contracted state. The control means may include means for operating the valve to control the rate of exit of the air.

The breathing system may include an adding means configured to regulate the flow of a separate gas such as nitrous oxide. The adding means increases or decreases the flow of gas into the breathing gas stream in accordance with a user control.

The breathing system may include stimulus provision means for providing stimulus to the patient, coupled to the control means. The stimulus provision means may be for providing one or more of: auditory, haptic and light stimulus visible to the patient. The control means is configured to control the adding by the adding means in dependence on inputs by the user in response to the stimuli. Where the stimulus is light stimulus, the stimulus may be to turn on of the light and off the light.

The patient may be informed before use of the system that the patient should respond to the stimulus with a predetermined operation of the user control.

The mounting means may comprise a patient airway interface, for example a mask, or a nasal clip.

The control means being configured to monitor for a response comprises being configured to: determine if a response is received from the user control within a predetermined period.

The control unit may be configured to control administration of the substance to the patient dependent on the result. The control means may be further configured to: measure time between providing the stimulus and receiving the response; and controlling administration of the substance based on the time.

According to a second aspect of the present invention, there is provided a method in a breathing system for a patient to inhale an analgesic, anaesthetic and/or sedative substance from, the breathing system comprising: a patient airway interface mountable on the patient so that inhaling and exhaling occur through the patient airway interface; adding means for adding the substance to gas to be inhaled, wherein the substance is provided in liquid or powder form, comprising vaporisation means for vaporising of the substance into the gas; a user control operable by the patient; a control means, the user control being coupled to the control means to provide inputs thereto in response to operation of the user control by the patient; wherein the control means is configured to store information indicative of a time of received inputs and/or of amounts of the substance added, wherein the method comprises: controlling the adding by the adding means dependent at least on: a most recently received input received from the user control, and the stored information indicative of the times of previously received inputs and/or of the amounts of the substance added further to the previously received inputs.

According to a third aspect of the present invention, there is provided a computer program product controlling adding of an analgesic, anaesthetic and/or sedative substance to air in a breathing system, which, when comprising computer program code which, when executed by processing apparatus, performs: storing of information indicative of a time of received inputs from a user control operable by a patient, and/or of amounts of the substance added; and controlling the adding by the adding means dependent at least on: a most recently received input received from the user control, and the stored information indicative of the times of previously received inputs and/or of the amounts of the substance added further to the previously received inputs.

The invention of the second and third aspect may implement any of the optional/preferred features and/or steps of the first aspect.

According to a fourth aspect of the present invention, there is provided a breathing system for a patient to inhale a substance from, comprising: a patient airway interface mountable on the patient so that inhaling and exhaling occur through the patient airway interface; adding means for adding the substance to gas to be inhaled, wherein the substance is provided in liquid or powder form, comprising vaporisation means for vaporising of the substance into the gas, wherein the vaporisation means is mounted in or on the patient airway interface so as to be mountable on a face of the patient; a user control operable by the patient; a control means, the user control being coupled to the control means to provide inputs thereto in response to operation of the user control by the patient, wherein the control means is configured to control adding by the adding means using the vaporisation means dependent at least on the inputs. The patient airway interface may be mountable on the face with one or more straps.

The vaporisation means may comprise a material located for gas to flow over or through it, and from which the substance can evaporate, wherein the adding means is configured to locate the substance on and/or through the material.

The breathing system may further comprise a housing mounted on or in the patient airway interface so as to be mountable on the face of the patient, wherein the housing retains the material and provides an inhalation path through or over the material.

The material may be planar. The breathing system may be configured for mounting of the material proximal the face of the patient. The inhalation path may pass perpendicularly with respect to the plane of the material.

The adding means may be mounted on or in the patient airway interface so as to be mountable on a face of the patient.

The vaporisation means may include a substance intake port and the adding means may comprise pumping means for pumping the substance to the material through the substance intake port.

The pumping means may be located physically spaced from the patient airway interface, optionally coupled to and co-located with the user control, and coupled to the substance intake port.

The control means may be coupled to and at least partially co-located with the user control. Alternatively, the control means is at least partially mounted on or in the patient airway interface so as to be mountable on a face of the patient.

According to a fifth aspect of the present invention, there is provided a system comprising: stimulus provision means; means for mounting the stimulus provision means onto a face of a patient; a user control operable by the patient; control means, the user control being coupled to the control means to provide inputs thereto in response to operation of the user control and the stimulus provision means being operatively coupled to the control means, wherein the control means is configured to: cause the stimulus provision means to provide the stimulus to the patient; and monitor for a response from the user control.

The stimulus provision means may be for providing one or more of: auditory, haptic and light stimulus visible to the patient. The control unit means may remove the stimulus after a response is received with the time period.

The patient may be informed before use of the system that the patient should respond to the stimulus with a predetermined operation of the user control. The substance may have sedative, anaesthetic and/or analgesic effect on the patient.

The mounting means may comprise a patient airway interface, for example a mask, or a nasal clip.

Where the stimulus is light stimulus, the stimulus may be to turn on of the light and off the light.

The control means being configured to monitor for a response comprises being configured to: determine if a response is received from the user control within a predetermined period.

The control unit may be configured to control administration of the substance to the patient dependent on the result. The amount of substance administrated may be reduced if a response is not received within the predetermined period.

The control means may be further configured to: measure time between providing the stimulus and receiving the response; and controlling administration of the substance based on the time.

According to a sixth aspect of the present invention, there is provided a substance administration system, comprising: stimulus provision means; a user control operable by the patient; control means, the user control being coupled to the control means to provide inputs thereto in response to operation of the user control and the stimulus provision means being operatively coupled to the control means, wherein the control means is configured to: cause the stimulus provision means to provide the stimulus to the patient; monitor for an input in response from the user control; control administration of the substance to the patient dependent at least in part on the response.

The stimulus provision means may be for providing one or more of: auditory, haptic and light stimulus visible to the patient. The control unit means may remove the stimulus after an input is received within the time period in response to the stimulus. Where the stimulus is light stimulus, the stimulus may be to turn on of the light and off the light.

The patient may be informed before use of the system that the patient should respond to the stimulus with a predetermined operation of the user control. The substance may have sedative, anaesthetic and/or analgesic effect on the patient.

The control means being configured to monitor for an input in response comprises being configured to: determine if a response is received from the user control within a predetermined period.

The control unit may be configured to control administration of the substance to the patient dependent on the result. The amount of substance administrated may be reduced if a response is not received within the predetermined period.

The control means may be further configured to: measure time between providing the stimulus and receiving the response; and controlling administration of the substance based on the time.

The control means may be configured to increase amount of the substance administered if an input by the user is not in response to the stimulus. For example, the input may be after input in response before a next stimulus is provided.

In accordance with a seventh aspect of the present invention, there is provided a system for administration of a substance having sedative, anaesthetic and/or analgesic effect on a patient, comprising: means for delivering the substance to the patient (e.g. a patient airway interface or intravenous delivery apparatus); adding means for controlling the amount of substance administered; a user control operable by the patient; a control means, the user control being coupled to the control means to provide inputs thereto in response to operation of the user control by the patient, the control means also being configured to control the adding of the substance by the adding means; stimulus provision means; wherein the control means is configured to: determine an adding rate, dependent on inputs by the user, where the rate of inputs is indicative of the patient being comfortable; further to determining of the adding rate, control the stimulus provision means to incite inputs from the user and to monitor consciousness level of the patient based on those inputs; determine, based on the monitoring indicating low consciousness level, to reduce the adding rate.

In accordance with an eighth aspect of the present invention, there is provided a method for administration of a substance having sedative, anaesthetic and/or analgesic effect on a patient, comprising: at a control means: determining a first value for an adding rate at which the substance is to be administered, dependent on rate of inputs by the user using a user control, where the rate of inputs is indicative of the patient being comfortable; further to determining the first value, controlling the stimulus provision means to incite inputs from the user and monitoring consciousness level of the patient based on those inputs; determining, based on the monitoring indicating low consciousness level, to reduce the adding rate; reducing the adding rate accordingly. There is also provided a computer program product comprising computer program instructions executable by processing means, for performing said method.

The control means is configured to determine the first value by: raising the adding rate in response to user inputs; lowering the adding rate in absence of user inputs; determining that the rate of user inputs corresponds to a predetermined rate over a predetermined period; determining the comfort value based on the one or more adding rates over that period. The stimulus provision means may be as set out for other aspects of the invention. The first value represents an adding rate at which the patient is comfortable.

### Brief Description of the Figures

Embodiments of the present invention will now be described, by way of example only, with reference to the accompanying drawings in which:
Figure 1 is an illustrative view of a breathing system in accordance with embodiments of the invention;
Figure 2 is an illustrative view of a breathing system in accordance with an embodiment, in which an adding unit includes gates that are operable together;
Figure 3A is an illustrative view of the breathing system of Figure 2;
Figure 3B is an illustrative view of a blocking member using the breathing system illustrated in Figure 3A;
Figures 4 and 5 illustrative air flow through the breathing system of Figure 3;
Figure 6 is an illustrative view of a user control of the breathing system;
Figure 7 is an illustrative view of a breathing system in accordance with another embodiment, in which an adding unit includes a pair of gates are individually operable;
Figure 8 is an illustrative view of a breathing system in accordance with another embodiment, wherein an adding unit comprises a hinged gate;
Figure 9 is an illustrative perspective view of an example breathing system when parts thereof are assembled together;
Figure 10 is an illustrative view of a breathing system in accordance with another embodiment, having an at least partially pneumatic control unit and user control;
Figure 11 is an illustrative view of a breathing system in accordance with another embodiment, including a spray device;
Figure 12 is an illustrative view of the spray device;
Figure 13 is an illustrative view of a breathing system in accordance with another embodiment including a pump,
Figure 14 is an illustrative perspective view of the breathing system of Figure 13;
Figure 15 is an illustrative view of a breathing system in accordance with another embodiment including a pump and multiple tubes;
Figure 16 is an illustrative perspective view of an example breathing system when parts thereof are assembled together;
Figures 17A to E are flow diagrams indicating steps in controlling administration of a substance;
Figure 18A and 18B are illustrative views of a breathing system in accordance with another embodiment;
Figure 18C is an exploded view of the breathing system of Figures 18A and 18B;
Figure 18D is a sectional view of the breathing system; and
Figure 18E indicates inhalation and exhalation paths through the breathing system.

### Detailed Description of Embodiments

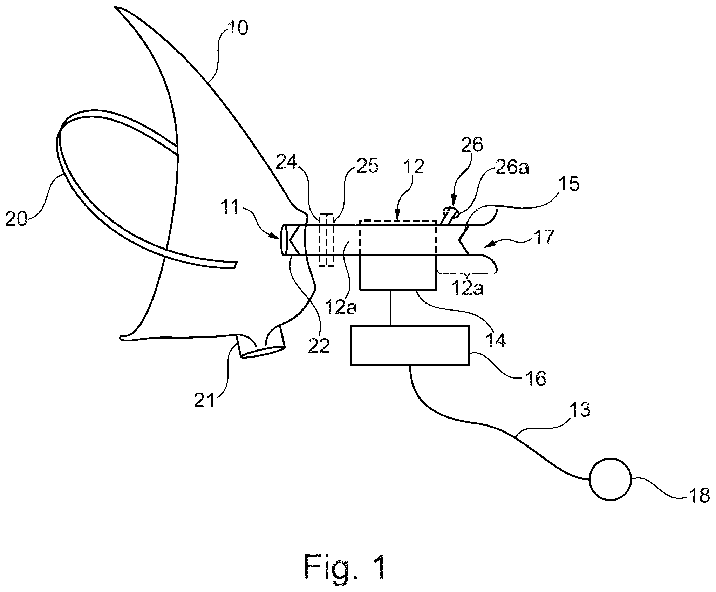

Breathing systems for mounting on a patient in accordance with embodiments are now described with reference to Figure 1. The breathing system comprises a patient airway interface (PAI) 10, an airway 12, an adding unit 14, a control unit 16 and a user control 18. The breathing system is for adding a substance to air to be inhaled, to administer the substance to the patient. Although not essential to embodiments, the substance may be a sedative, an analgesic or an anaesthetic. For example, the substance may be methoxyflurane, adrenaline or salbutamol, ketamine or any other inhalable drug compound or volatile anaesthetic agent or gas.

The PAI 10 is in the form of a mask 10. The mask 10 has an opening (not shown, but location indicated at 11), to which a first end of the airway 12 is fixedly and sealingly connected, so that air can be inhaled through the airway 12 into the mask 10 and then into the lungs of the patient. The mask 10 also has a strap 20 for mounting of the mask 10 around the mouth and nose of the patient.

A second end of the airway 12 forms an opening 17 to the environment, allowing entrainment of air from the environment. The adding unit 14 is located to add the substance to gas in the breathing system between the openings 11, 17.

In general herein, "air" is referred to as the gas that is drawn into the breathing system from the environment, while the term "gas" is used to refer to gas within the breathing system even where the gas inhaled from the breathing system is solely air. The term "adding rate" is used herein to refer to the rate that the adding unit adds the substance to gas to be inhaled. In different embodiments the adding rate may be changed by operation of one or more valves allowing gas with the substance in to be inhaled and/or by changing a rate at which the substance is vaporised. It will appreciated that the "administration rate" closely corresponds, with a time lag for the gas to which substance has been added to be inhaled.

In some variant embodiments, the breathing system includes an inlet port 26 connected to the airway 12 so that gas from a source other than the environment can be inhaled, such as oxygen or oxygenated air. The source may be a cannister or a wall outlet of the kind commonly found in hospitals to which the inlet port 26 can be connected. The inlet port 26 is not essential to embodiments. A closure 26a is provided for when the port is not in use. In a variant, a plurality of such inlet airways may be provided enabling connection of a plurality of sources of gas each being different, for example nitrous oxide and oxygen, such that gases from those sources may be inhaled.

In such variant embodiments, the control unit 16 may be connectable to one or more regulators that regulates flow of gas from the one or more other sources. The control unit 16 may be configured to control flow rate from such one or more other source. The control unit 16 may also be connected to a regulator that regulates flow of air into the breathing system at the opening 17. For example, the control unit 16 may be configured to control relative amounts of environmental air, oxygen and/or nitrous oxide that are entrained into the breathing system. This facilitates ensuring that analgesia and oxygen requirements are met. In another variant, the airway 12 may not open to the environment at opening 17 and may connect exclusively to a source of gas that is other than environmental air. For example, the second end of the airway 12 may connect to a canister or such a wall outlet.

The breathing system is such that the patient can breathe through it, to inhale and exhale consecutively and repetitively. The mask 10 includes an expiratory valve 21 and an inspiratory valve 22. The inspiratory valve 22 is a one-way valve located in the mask 10 to prevent flow of exhaled air through the adding unit 14 but to permit flow from the airway 12 into the mask 10. The expiratory valve 21 is a one-way valve enabling exhaled gas to exit the mask 10 to the environment but preventing ambient air from entering the breathing system. The airway 12 also includes a one-way valve 15 located between the second end of the airway 12 and a region of the airway 12 where the substance is added, to prevent gas in the airway 12 with substance in from exiting the breathing system through the opening 17, but enabling entrainment of air. In some embodiments, the inspiratory valve 22 may be unnecessary in view of the one-way valve 15. The inspiratory and expiratory valves 21, 22 may be provided each as a unidirectional silicone rubber flap (mushroom valve), for example. The inspiratory and expiratory valves 21, 22 may be built into the mask 10 as a single valve assembly. Various valve design providing the functionality of the inspiratory and expiratory valves 21, 22 are known in the art and embodiments are not limited to any particular design. The breathing system may also include a filter over the expiratory valve 21 to prevent any substance from being passed in surrounding air and disseminated.

In variant embodiments, the inspiratory valve can be located across the airway 12 rather than being part of the mask 10. In this case, the expiratory valve may be included in the airway 12, in a region of the airway between the mask 10 and the inspiratory valve 22. In other variant embodiments, the expiratory valve 21 and the one-way valve 15 may be absent; in such embodiments exhaled air may exit the breathing system through the airway 12. This may be appropriate in embodiments where exhaled gas does not include substance that needs filtering.

In variant embodiments, the mask 10 may be replaced with another type of PAI. The PAI may be in the form of a nasal mask or an invasive device such as a supraglottic airway, for example. The nasal mask may in particular be used in the field of dentistry. Breathing through the mouth may be prevented by means known in the field. Embodiments of the invention are not limited to any particular form of PAI that acts as a conduit for all inhaled and exhaled respiratory gasses. The breathing system as a whole enables and requires the patient to repetitively inhale and exhale through the breathing system.

The adding unit 14 is configured to add the substance to gas in the breathing system through vaporisation, particularly through evaporation. In some embodiments, the adding unit 14 includes a piece of material, for example cotton, on or through which the substance is dispersed to increase the surface area of the substance, thereby to facilitate evaporation into the gas.

Embodiments are not limited to any particular kind of adding unit 14. Different examples of adding units 14 are given in embodiments described below. The adding unit 14 may be arranged to add the substance to gas within the airway 12, such that entrained gas collects the substance as it flows through, or the adding unit 14 may add the substance to gas outside the airway 12 and include a flow control for controlling whether gas without the substance is inhaled from the airway 12 or whether gas with the substance is inhaled where the substance was added outside the airway 12 or whether a mixture is inhaled. The gas with the substance in may flow into the mask 10 via the same opening 11 as the gas without the substance, or in alternative embodiments the gases may flow in each through a respective opening in the mask 10. In embodiments where the substance is added to the gas outside of the airway 12, the gas to which the substance is added may flow in through a different intake than the opening 17.

In some embodiments, the adding unit 14 can be controlled to control rate of evaporation, for example by controlling an area from which the substance evaporates and/or the temperature of the substance and/or the rate at which substance is located on the material. It is to be noted however that, while a maximum amount of the substance that is added, in total and/or over particular time periods, may need to be carefully controlled to prevent overdose, accurate control of the amounts to ensure that enough is administered is of lesser importance, since the patient may operate the user control to administer more if appropriate.

The control unit 16 is coupled to the adding unit 14 to control the adding unit 14. The control unit 16 is also configured to receive input from the user control 18. In some embodiments, the input may be in the form of electrical trigger signals and in others mechanical or pneumatic trigger input. Where the control unit 16 controls the adding unit 14 electronically, the control unit 16 provides control signals to the adding unit 14. These signals may be start and stop signals for actuators to open and close valves in embodiments where operation of flow control is binary. These signals may set and update adding rates where the rate of adding is controllable in a non-binary manner.

The user control 18 is operable by the patient to provide the input to the control unit 16. The patient operates the user control 18 when the patient wishes more of the substance to be administered. For example, where the substance is an analgesic agent, the input may be indicative of a desire for pain relief. Embodiments are not limited to where the patient controls the user control 18; an attendant operator may control the user control 18, for example.

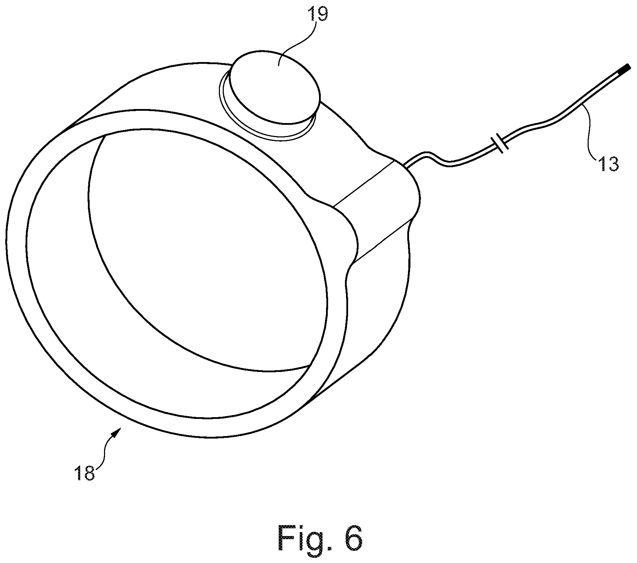

Embodiments of the invention are not limited to the user control 18 being in any particular form. In some embodiments, the user control 18 is readily usable by the patient. The user control 18 may be operable by finger action of the patient. The user control 18 may be mountable on the patient's hand, for example with a strap, or on a finger of the patient. In some embodiments, the user control 18 is physically spaced from the control unit 16 whereas in others the user control 18 is located together with the control unit. Referring to Figure 6 by way of example, the user control 18 is a ring on which a pressure-sensitive button 19 is located. The user control 18 is coupled to the control unit 16 by the cable 13.

In some embodiments, at least one part of the breathing system other than the mask 10 may be provided physically separate to the mask 10. For example, where all parts other than the mask 10 are provided separately, the first end of airway 12 may be attachable to the mask 10 around the opening 11 in a sealing manner, so that gas drawn through the airway 12 can pass through the opening into the mask 10 without egress to the environment. A plurality of masks 10 may be provided of different sizes, each attachable to the airway 12. Thus, a mask 10 of appropriate size may advantageously be selected for a particular patient.

In such alternative embodiments, in order to enable attachment, the mask 10 includes a first connector piece 24 and the airway 12 includes a second connector piece 25. These are indicated using broken lines in Figure 1 to indicate that they may be present in some embodiments but not others. The first connector piece 24 is at the opening to the mask 10 and the second connector piece 25 is located at the first end of the airway 12. The first and second connector pieces 24, 25 are annular and threaded so as to be connectable in a relative twist action. In variant embodiments, the first and second connector pieces 20, 24 are otherwise configured to connect the mask 10 and the first airway 22 in a sealing manner so that gas can be drawn from the flow control 14 without egress to the environment.

The control unit 16 is configured to control the adding unit 14 dependent on input from the user control 18. The inputs are discrete and the control unit 16 may be configured to cause an amount of the substance to be added to the air in the airway 12 in response to an input from the user control 16. Alternatively inputs may be continuously provided for example by continued depression of a button by the patient.

The control unit 16 may be configured to cause the adding unit 14 to add the substance into gas to be inhaled at a background rate, the background rate being low relative to a maximum amount that can be safely administered to the patient. The background rate is a minimum rate that is administered regardless of input caused by the patient.

In embodiments in which the control unit 16 is an electronic control unit, the control unit 16 comprises a battery and a microcontroller. The microcontroller comprises a processor, a memory, a clock, and input/output interfaces operatively connected by a bus. The control unit may have additional components. Computer program codes including operational algorithms are stored in the memory which, when executed by the processor, result in the microcontroller performing the steps described herein. Alternatively, functionality of the control unit 16 may be implemented with dedicated hardware, or a mixture of hardware and software. The user device 18 is in such embodiments configured to provide input as electronic trigger signals to the control unit 16 and the adding unit 14 is configured to be electronically controlled. The control unit is configured to receive and to process the received trigger signals in accordance with stored algorithms and control the adding unit 14 accordingly. The trigger signal is an indication that more of the substance is wanted by the patient. Embodiments are not limited to any particular format that the signals may take. In an alternative embodiment input may be mechanical or pneumatic and the control unit may control the amount added dependent on the information received from one or more sensors (e.g. control unit 74).

The control unit 16 is also configured to prevent an excessive amount of the substance being inhaled, particularly due to the patient operating the user control 18 too frequently to signal that more of the substance is to be inhaled. The algorithms include rules preventing this. For example, a maximum rate may be configured that cannot be exceeded. Alternatively, a maximum administration time interval may be enforced.

Embodiments of the invention are not limited to the airway 12 having any particular length, lengthwise shape or cross-sectional size or shape, provided the dimensions enable air flow and unless the embodiments require otherwise.

In operation, the breathing system enables the patient to breathe through it and also administers the substance. First, the mask 10 is fitted to the patient, covering the nose and mouth. In alternative embodiments, another type of PAI is fitted. The patient is then compelled to breathe through the breathing system, with repetitive inhalations and exhalations. An operator may be required to turn on the breathing system. The control unit 10 may control the adding unit 14 to add the substance to the air in the airway 12, such that the substance is administered to the patient at the background rate. The patient may operate the user control 18 to provide input to the control unit 16. When such input is received by the control unit 16, the control unit 16 causes the adding unit 14 to add more of the substance to entrained gas.

Embodiments described below can be considered to be possible implementations of the generalised embodiments described above. Like parts may be denoted by like reference numerals in the following, but are not always. Parts that differ to those described in relation to Figure 1, but perform a same or similar function may be given the same reference numeral incremented by 100 or a multiple thereof, but are not always.

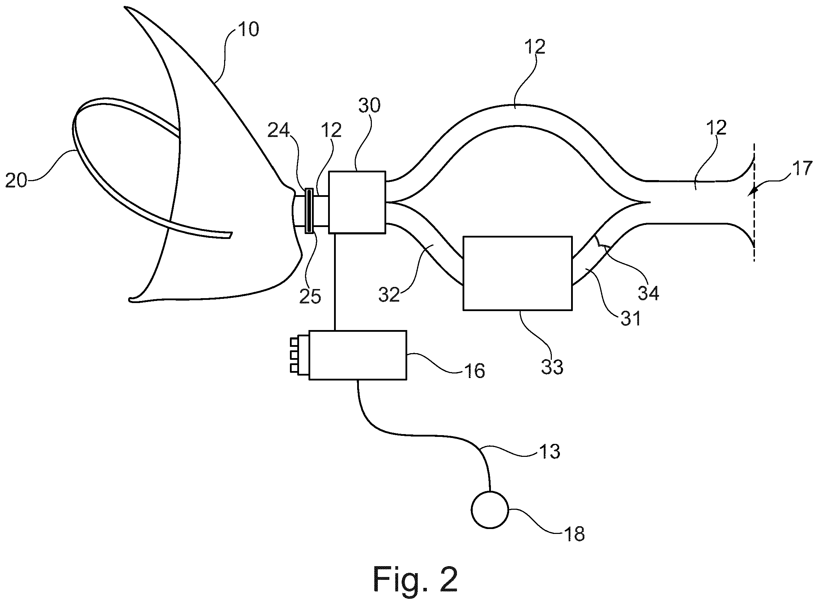

Referring to Figure 2, in an embodiment, the adding unit 14 comprises a flow control 30, a first connector airway 31, a second connector airway 32 and a vaporisation unit 33. The vaporisation unit 33 is connected to the airway 12 by the first connector airway 31. In this embodiment, a one-way valve 115 is located in the first connector airway 31 to prevent gas from within the vaporisation unit 33, which typically has the substance in it, from flowing back into the airway 12. Alternatively, the one-way valve 115 may be part of the vaporisation unit 33. The second connector airway 32 connects the vaporisation unit 33 to the flow control 30, so that gas can flow from the vaporisation unit 33 to the flow control 30. As the skilled person will appreciate, the first and second connector airways 31, 32 may be short and may in practice be an intake and outlet to the vaporisation unit 33. Also, in a variant embodiment, the first connector airway 31 may not connect to the airway 12, but instead open to the environment so that environmental air can be entrained.

The control unit 16 is coupled to the flow control 30 to control operation thereof. The flow control 30 provides a airflow junction in the airway 12 with gates. In some embodiments, the gates are controllable to be in either a first configuration or a second configuration (and not between the two configurations). In the first configuration, a first gate across an end of the second connector airway 32 is closed, completely blocking gas flow from the vaporisation unit 33, and the second gate across the airway 12 is open, so that only air from the environment can be drawn into the mask 10. In the second configuration, the first gate is open, permitting gas flow from the vaporisation unit 33, and the second gate is closed, preventing flow of air through the airway 12. Such embodiments permit binary control of the air flow. In other embodiments, both the first and second gates may be partially open, as well as wholly open or closed, so that air that has passed through the vaporisation unit 33 is mixed with air that has not. Although not essential to all embodiments, the flow control 30 may (in binary or non-binary embodiments) be configured or controllable to reduce air flow from one of the two air flow paths in inverse proportion to the extent to which air flow is permitted on the other of the two air flow paths. This results in resistance to inhalation when breathing through the breathing system remaining substantially unchanged regardless of the path of air through the breathing system.

The flow control 30 includes a biasing means to bias the flow control 30 to its first configuration. Thus, in the event of physical failure the flow control 30 is configured to enable an uninterrupted flow of air from the environment without addition of any substance from the vaporisation unit 12.

The vaporisation unit 33 includes a port to enable pouring or squirting in of the substance. The vaporisation unit 33 may also define a vaporisation chamber 35 into which the substance is poured or squirted providing a reservoir. Such a port includes a lid to close it, and the vaporisation unit 33 may thereby be sealed to prevent the substance evaporating or spilling out.

In a variant embodiment, the vaporisation unit may be configured to enable insertion or attachment of a capsule containing the reservoir via a port. In this case the capsule is openable to enable the vaporisable substance to flow out. The capsule may in an embodiment include an opening with a closure that is broken open by a portion of a lid for the port in an action of closing the port with the lid.

Embodiments are not limited to any particular design of vaporisation unit, nor any particular way in which the vaporisable substance is provided to the vaporisable unit 35. In some embodiments, the vaporisable unit 33 may not permit provision of new substance, but instead may be only for use with an amount of the substance located therein during manufacture. In this case the vaporisation unit may be replaceable in the breathing system, or the whole breathing system is disposed of after use.

In operation of the breathing system, the control unit 16 controls the flow control 30 to control the amount of gas passing through the vaporisation unit 33 that is inhaled. In variant embodiments, the control unit 16 may also be coupled to the vaporisation unit 33, where the vaporisation unit is such that rate of evaporation of the substance is controllable.

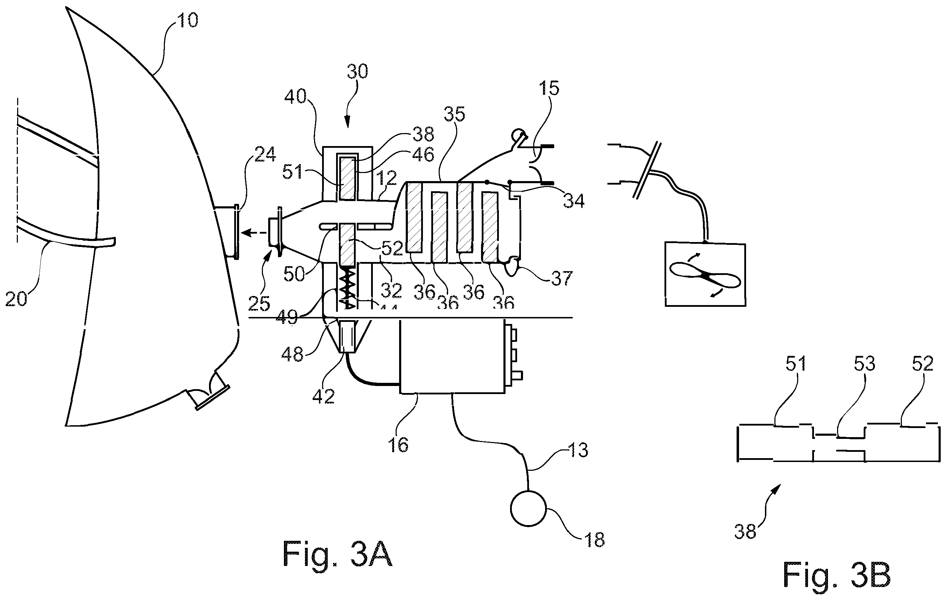

Referring also to Figure 3A, which is an example of the embodiment described with reference to Figure 2, the vaporisation unit 33 comprises a vaporisation chamber 35 and a material comprises a wick 36 inside the vaporisation chamber 35. The wick 36 has a structure configured to expose a large surface area to passing gas in view of the volume of the wick, for example by having a labyrinthine or porous structure, thereby to facilitate evaporation of the substance. The wick 36 is configured to disperse the substance through it. A lid 37 provides a closure for a port through which the substance can be added to the chamber 35 by pouring or squirting, as mentioned above.

The flow control 30 comprises an actuator assembly, an airway blocking member 38 and a housing 40. The actuator assembly comprises an actuator 42 and the biasing means in the form of a resilient member 44, for example a spring. The actuator 42 is coupled to the control unit 16. The airway 12 and the second airway connector 32 each have a pair of opposing apertures therein, which are all aligned. The housing 40 includes a first airway projection 46 over a first of the apertures in the airway 12. The first airway projection 46 provides a first recess therein extending from the interior of the airway 12. The housing 40 also includes a second airway projection 49 over a first of the aperture in the second airway connector 32. The second airway projection 49 provides a second recess therein extending from the interior of the second airway connector 32. The housing 40 also provides a connecting channel 50 connecting a second of the apertures in the airway 12 and a second of the apertures in the second connector airway 32. The first and second recesses and the connecting channel 50 are aligned. The first airway connector 31 is in this embodiment an inlet to the vaporisation chamber 35 from the airway 12, the one-way valve 34 being provided at the inlet.

The blocking member 38 is disposed in the housing 40 to be slidable in the first and second recesses and the connecting channel 50, and across the airway 12 and the second airway connector 32, between first and second positions. The control unit 16 is arranged to control the position of the blocking member 38. In the first position gas flow from the vaporisation unit 33 is wholly blocked and the flow of air through along the airway 12 into the mask 10 is permitted. In the second position, gas flow through the vaporisation unit 33 is permitted and flow through the airway 12 is blocked. The first and second airway projections 46, 49 and a portion of the housing 40 providing the connecting channel 50 respectively seal the first apertures in the airway 12 and the first airway connector 31, and the second apertures, to prevent egress of gas from the breathing system. When the blocking member 38 is disposed between the first and second positions, gas may be entrained both through the airway 12 and also through the vaporisation unit 33.

In greater detail, referring also to Figure 3B, the blocking member 38 comprises a first gate 51, a second gate 52 and a bridge 53 connecting the first and second gates 51, 52. When the blocking member 38 is in the first position, the first gate 51 is located in the first recess and the second gate 52 blocks flow of gas through the second connector airway 32. When the blocking member 38 is in the second position, the first gate 51 blocks the airway 12 and the second gate 52 is located in the second recess. The bridge is moveable lengthwise in the connecting channel 50, while also sealing the channel 50 to prevent gas flow in the channel 50.

In a variant design, the airway 12 and the second connector airway 32 may share a wall such that the connecting channel 50 is an opening between the second connector airway 32 and the airway 12.

The actuator 42 is controllable to move the blocking member 38 between the first and second positions. The actuator 40 is a piston and cylinder type actuator. A piston 48 thereof extends through an aperture in the second airway projection 46 and also seals the aperture to prevent egress of gas. A distal end of the piston 48 is fixed to the blocking member 38 to control the position of the blocking member 38. The resilient member 44 is disposed to bias the blocking member 38 into the first position. The blocking member 38, the first and second recesses and the connecting channel 50 are shaped so that, to the extent that the gas flow through the airway 12 is prevented, air flow via the vaporisation unit 33 is permitted, and vice versa. Thus, resistance to inhalation is substantially the same regardless of the position of the blocking member 38.

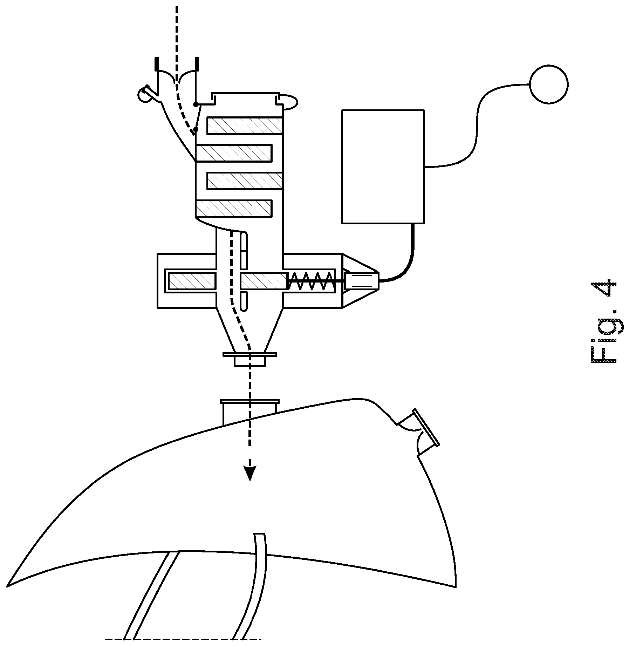

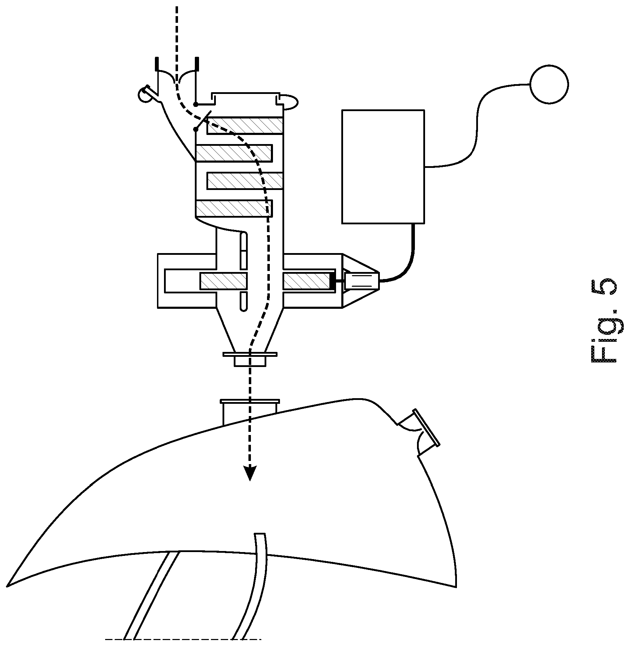

The flow control 30 is in the first and second configurations when the blocking member 38 is in the first and second positions. An arrow in Figure 4 illustrates air flow during inhalation when the flow control 30 is in the first configuration. Another arrow in Figure 5 illustrates air flow during inhalation when the flow control is in the second configuration.

In use, the control unit 16 controls the actuation assembly to control the position of the blocking member 38 dependent on received trigger signals and on the stored algorithms. The substance may also be administered to the patient at at least the background rate on each inhalation and the flow control 14 may control the position of the blocking member 38 so that some flow through both the airway 12 and from the vaporisation unit 33 is permitted.

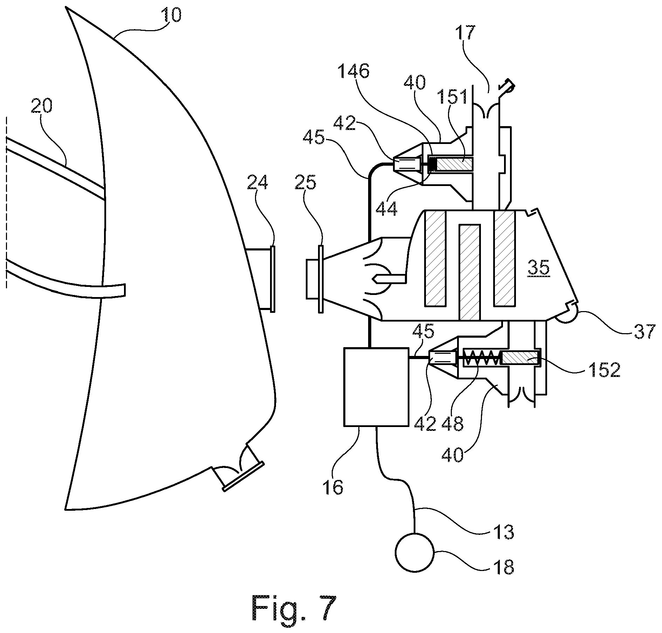

Embodiments of the invention are not limited to any particular manner in which the flow control 30 is implemented. Referring to Figure 7, in another embodiment, the flow control 30 includes first and second actuator assemblies respectively configured to move first and second gates 151, 152 to control gas flow through the airway 12 and the vaporisation unit 33. Also in this embodiment, the first connector airway 132 is open to environmental air. In a variant embodiment, the first connector airway 132 may join with the airway 12 and include a single opening to the environment, like opening 17 described above.

Each of the actuator assemblies are configured in the same way as the actuator assembly described with reference to Figure 3A and, accordingly, include a housing 40, an actuator 42 including a piston 48, and a resilient member 44. Each actuator assembly is coupled to the control unit 16 with a respective cable 45.

The first gate 151 is moveable by the first actuator assembly between a first position in which the first gate 151 is located in a recess in a first airway projection 146 extending from the airway 12, and a second position in which the gate 151 blocks the airway 12.

The second gate 152 is moveable by the second actuator assembly between a first position in which the second gate 152 blocks the first connector airway 132, thereby preventing air from entering the vaporisation chamber 35, and a second position in which the second gate 152 permits environmental air to be drawn into the vaporisation chamber 35. Notably, the second gate 152 is configured for blocking of air from entering the vaporisation unit 12 in contrast to the embodiment illustrated in Figures 3A, 3B, 4 and 5 in which exit of gas from the vaporisation unit 12 is blocked. Unlike the embodiments illustrated in Figures 3A, 3B, 4 and 5, the first and second gates 151 152 can be moved independently of one another.

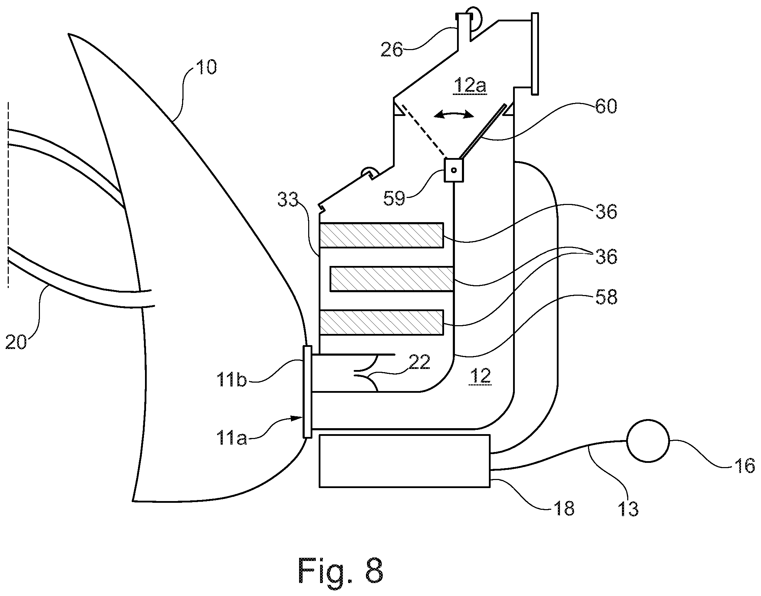

Referring to Figure 8, in a breathing system according to another embodiment, the flow control 30 comprises gate 60 that can be moved between a first position or a second position about a hinge 59. In the first position, illustrated in Figure 8, air must flow through the vaporisation chamber 35 of the vaporisation unit 33 during inhalation, and flow of air through the airway 12 is blocked. In the second position, illustrated with dashed lines in Figure 8, the gate 60 prevents flow of air through the vaporisation unit 33 and air must instead pass through the airway 12. Although not essential, the airway 12 and the vaporisation unit of this breathing system includes a shared wall partitioning them, the hinge 59 being located at an end of the shared wall 58.

In this embodiment, the mask 10 includes separate openings 11a, 11b to sealingly connect respectively to the first end of the airway 12 and the second connector airway 32. The breathing system does not include an expiratory valve 21. An inspiratory valve 22, that is, a one-way valve 22 is located in the second connector airway 32 to prevent exhaled air from entering the vaporisation unit 33. Exhaled air flows through the airway 12 to exit the breathing system. The gate 60 is mounted to lift, if closed, when the pressure due to exhaled air in the airway 12 exceeds the pressure on distal portion of the airway 12. Exhaled air can then exit the apparatus through the airway 12. In an alternative embodiment, the mask 10 includes the expiratory valve 21 for exhaled gas to pass through. In this case the airway 12 preferably includes a one-way valve (not shown) to prevent exhaled air from flowing through.

Embodiments are not limited to any particular form of gate. For example the gate may be configured to narrow or close the airway 12 and/or the second connector airway 32, like an electronically actuatable shutter or iris of a camera.

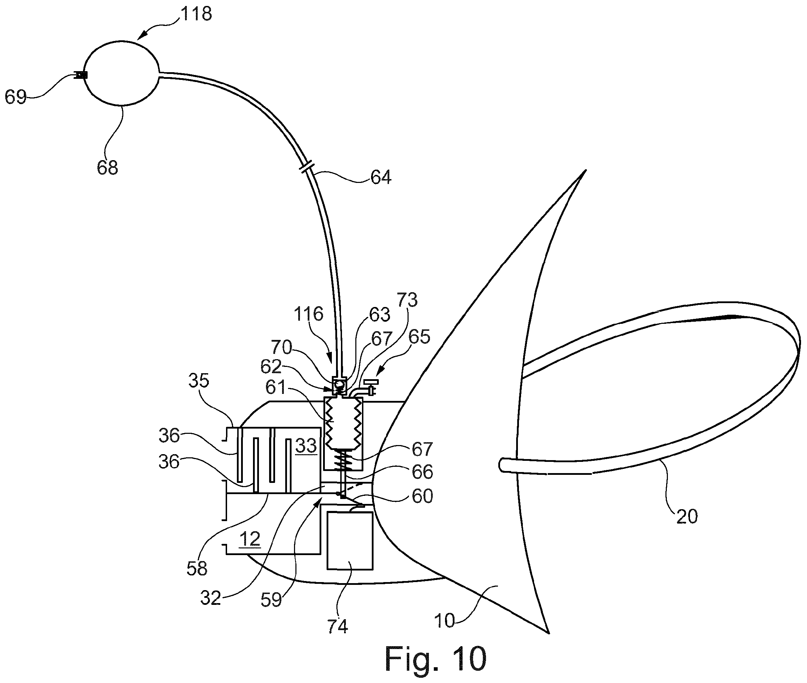

Referring to Figure 10, in another embodiment, the vaporisation chamber 35 has an inlet for intake of environmental air. Like the embodiment described with reference to Figure 8, a gate 60 can be pivoted from a first position to a second position about a hinge 59. In the second position, illustrated in Figure 10, air must flow through the vaporisation unit 33 during inhalation, and flow of air through the airway 12 is blocked. In the first position, illustrated with a dashed line in Figure 10, the gate 60 prevents flow of air through the vaporisation unit 33 and air must instead pass through the airway 12.

The control unit 116 and the user control 18 in this embodiment are not electric, but are instead pneumatic. The control unit 116 comprises a housing 140, a bellows 61, a check valve 62, a cylinder 63, a tube 64 and a release valve 65. A flow control 130 comprises an actuator rod 66 and a biasing means in the form of a resilient member 67, for example a spring.

The user control 118 comprises a hollow squeezable bulbous portion 68. The tube 64 is connected at a first end thereof to the bulbous portion 68 such that the interior of the bulbous portion 68 and the interior of the tube are in fluid communication and sealed with respect to the environment. The user control 118 also includes a one-way valve 69 in a wall of the bulbous portion 68 arranged so that air from the environment can enter the bulbous portion 68 when the pressure in the bulbous portion 68 is lower than the environmental pressure.

The tube 64 is connected at a second end thereof to an opening into the cylinder 63 such that the interior of the cylinder 63 and the interior of the tube 64 are sealed with respect to the environment. The check valve 62 is arranged so that, when air pressure in the tube 64 exceeds air pressure in the cylinder 63 by a threshold pressure, the check valve 62 permits air from within the tube 64 to pass into the cylinder 63.

The check valve 62 comprises a ball 70, a resilient member 71 in the form of a spring, and an annular piece of rubber 72 around the aperture into the cylinder 63. The ball 70 is biased by the resilient member 71 into an aperture through the rubber piece 72, thereby to close the aperture and block movement of air between the interior of the cylinder 63 and the interior of the tube 64. When the air pressure in the tube 64 exceeds the air pressure in the cylinder 63 by greater than the threshold pressure, the ball 70 is pushed away from the rubber piece 72 against the resilient member 71, allowing passage of air from the interior of the tube 64 into the cylinder 63. When the pressure difference drops below the threshold pressure, the resilient member 71 pushes the ball 70 back into the aperture of the rubber piece. Variant embodiments do not require use of a check valve 62 of this particular type; other kinds of check valve exceed. The check valve 62 may instead be provided as a one-way valve, where threshold pressure is very low.

The bellows 61, located in the housing 140, provides an interior space connected to the interior of the cylinder 63. An outlet 73 from the bellows 61 is connected to the release valve 65. The interior of the bellows 63 is otherwise sealed with respect to the environment. The actuator rod 66 extends through a hole in the housing 140 to partially project from the housing 140. A first end of the actuator rod 66 is fixed to the bellows 61 and a second end thereof is fixed to the gate 60. The bellows 61 is expandable in the housing 140 in a direction of the length of the actuator rod 66 to further project the actuator rod 66 from the housing 140. The resilient member 67 is located between the housing 140 and the bellows 61 to bias against expansion of the bellows 61, thus urging contraction of the bellows 61. Contraction of the bellows 61 draws the actuator rod 66 further into the housing 140.

Extension of the actuator rod 66 determines position of the gate 60. The actuator rod 66 is extendable to a maximum extended position in which the gate 60 blocks the airway 12, and retractable to a minimum extended position in which the gate 60 closes the second connector airway 32.

The release valve 65 includes a control, for example a tap or gate, to control the rate of release of air from the interior of the bellows 61. The control may be manually adjustable to adjust the rate at which the air flows through the valve 65, out of the bellows. This controls the length of time that gas may be inhaled from the vaporisation unit 30 without further pumping from the patient.

Although not essential, in some embodiments the control unit 116 also includes a microcontroller and battery, and a position sensor 74. The position sensor 74 is arranged to monitor when the gate 60 is open and closed and provide such information to the control unit 116. The position sensor may include an electrical contact on the gate 60, which touches another electrical contact when the gate 60 is open and yet another electrical contact when the gate 60 is closed, making and breaking a circuit. The control unit 16 is configured to estimate amount of drug added dependent on the length of time that the gate 60 is open for. Further, the control unit may be coupled to the control of the release valve 65 for automated operation thereof (i.e. control the tap or gate thereof). The control unit is configured to operate the control to allow faster flow from the interior of the bellows 61 to reduce the amount of the substance added to air to be inhaled. Where the valve 65 is sufficiently open, no further substance is added. The control unit is configured to operate the control to allow slower flow from the interior of the bellows 61 to increase the amount of the substance added to the air to be inhaled.

The control unit 116 is configured to control valve 65 to control the amount of substance added, in a similar way to how the control unit 16 may control one or more actuators in other embodiments.

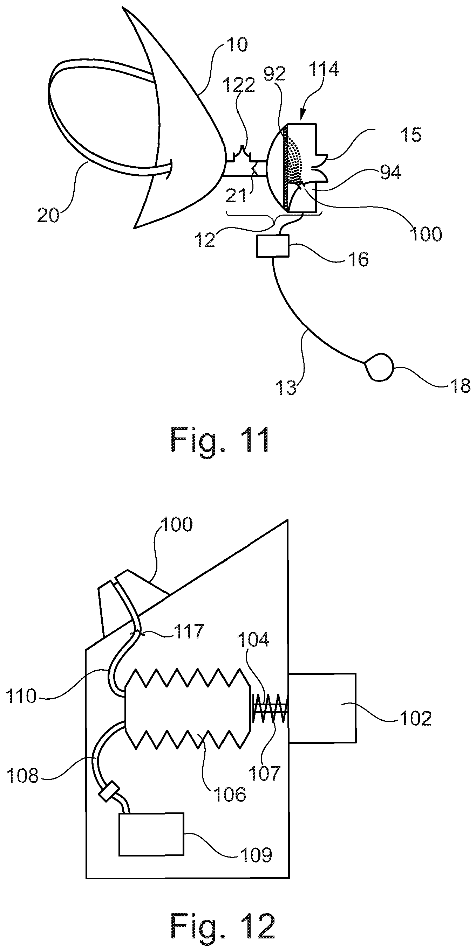

Referring to Figure 11, a breathing system in accordance with another embodiment comprises an alternative adding unit 114 having an alternative vaporisation unit which is provided across the airway 12. Air can only be entrained into the breathing system in a single path. The vaporisation unit comprises a piece of material 92 and a spray device 94. A section of the airway 12 is enlarged relative to a rest of the airway 12, and this section serves as a housing 90 of the vaporisation unit. The material 92 spans an interior of the housing 90. The breathing system is arranged so that, when a patient inhales, air is drawn in through the opening of the housing 90, passes through the material 92 into the airway 12 and then into the mask 10 and into the patient. The breathing system is arranged so that exhaled air exits the breathing system through the expiration valve 122. The spray device 94 includes a reservoir (not shown), containing the substance, a nozzle 100 directed towards the material 92 and means for propelling the substance through the nozzle 100 onto the material 92. The control unit 16 is coupled to the means for propelling the substance to control timing and amount of the substance sprayed on the material 92. The amount may be controlled by the length of the time over which it is sprayed. Alternatively, in embodiments the spray device 94 may be configured so that the rate of spraying is controllable and in this case the amount may be controlled by controlling the rate, additionally or alternatively to the length of time. The substance is sprayed as droplets. The droplets evaporate into air drawn in through the opening 15 that passes through the material 92.

The material 92 is preferably absorbent, although this is inessential to all embodiments. The material 92 is arranged to span the cross-section of the housing 90 so that entrained gas must pass through the material 92, while also presenting low resistance to inhalation. The material 92 may span wholly across the housing 90, or only in part. Embodiments are not limited to any particular disposition of the material 92 in the housing 90, provided sufficient evaporation of the substance into the passing air takes place for effective administration and inhalation is not difficult for the patient. The material 92 may, for example, be in the form of a piece of gauze or fabric (e.g. cotton) pad. Embodiments are not limited to any particular form.

In use, the control unit 16 provides control signals to the spray device 94. In response to such a signal, the spray device sprays a bolus of the substance onto the material 92. The control unit 16 can send the control signals further to receiving a trigger signal from the user control 18 and/or in response to the control unit 16 determining that an amount of the substance is to be added at a background rate. When the patient inhales, air is entrained through the opening, via the one-way valve 98, through the housing 90 and the material 92, into the mask 10 and from there into the patient's lungs, where the substance may be absorbed. In absence of operation of the spray device 94, breathing gradually removes the substance from the material 92 by evaporation, so that eventually only air is breathed.

Embodiments are not limited to any particular form of spray device 94. Referring to Figure 12, an example of how such a spray device may function is described. As well as including the reservoir, indicated at 109, and the nozzle 100, the spray device 94 includes an actuation unit 102 including an extendable and retractable piston 104, a bellows 106, a biasing means in the form of a resilient member 107, for example a spring, and first and second channels 108, 110. The first channel 108 connects the reservoir to an interior of the bellows 106 in a sealing manner. A one-way valve 117 is located in the second channel 110 to prevent backflow. The second channel 110 connects the interior of the bellows with the nozzle 100. The actuator unit 102 is coupled to the control unit 16. The actuation unit 102 is arranged to respond to control signals from the control unit 16 by extending or retracting the piston 104. A remote end of the piston 104 is fixed to the bellows 106. The bellows 106 can be expanded and compressed between a maximum expanded position and a minimum expanded position by retraction and extension of the piston 104. The resilient member 107 is arranged to bias the bellows 106 into the maximum expanded position. In use, when the actuation unit 102 receives a control signal from the control unit 16, the actuation unit 102 extends the piston 104. Extension of the piston 104 compresses the bellows 106, pushing the substance from the interior of the bellows through the second channel 110 towards the nozzle 100. As already mentioned, the nozzle 100 is arranged to spray the pressurised substance onto the material 92. Retraction of piston 104, whether due to action of the resilient member 107 and/or the actuation unit 102, draws the substance into the interior of the bellows 106 from the reservoir 99 through the first channel 108.

This example of the spray device may be varied in alternative embodiments. Various appropriate ways of implementing a spray device actuatable by the control unit 16 will be available to persons skilled in the art. For example, an ultrasonic transducer such as a piezoelectric element may be used to nebulise liquid drug agent. Such airborne particles may travel down the airway lodging in material 92 whereby vaporisation may occur.

In an alternative embodiment, the spray device of Figure 11 can be modified to be pneumatically (or mechanically) actuated rather than electronically activated. To this end, the spray device of Figure 11 can be modified to include the control unit 116 and user device 118 of Figure 10, such that the actuation rod 66 is fixed to the bellows 106 rather to the gate 66.

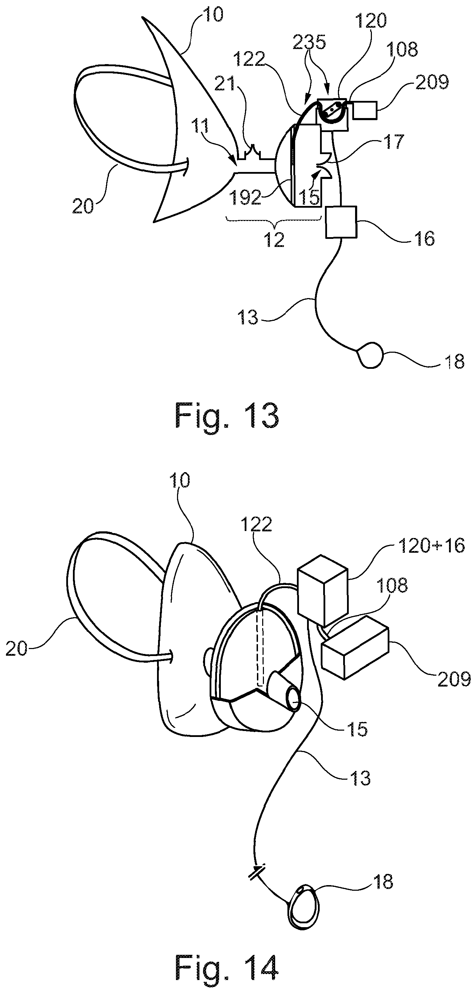

Referring to Figures 13 and 14, a breathing system in accordance with another embodiment is like the breathing system described in relation to Figure 11, but the adding unit 14 has an alternative vaporisation unit 235. The vaporisation unit 235 comprises a material 192, a reservoir 209 containing the substance, a peristaltic pump 120 and a tube 122. The tube 122 is positioned to open onto the material 192. The material 192 is such as to wick the substance deposited onto it from the tube 122 away by capillary action so as to be absorbed and distributed over the material 192. The peristaltic pump 120 is operable to drive flow of the substance in the tube 122 in response to control signals from the control unit 16. The material 192 may, for example, be in the form of a piece of gauze or fabric (e.g. cotton) pad. The material 190 may be a meshwork.

Embodiments are not limited to any particular form of the material 192, provided air may pass through it and the substance evaporates from it into the air. The material 192 may be essentially flat and extend across the housing 90, or alternatively may extend lengthwise in the housing 90. The material 192 may be the same as the wick 36 (e.g. cotton) described above so that dispersal of the substance through the material 192 occurs due to characteristics of the material of the material 192.

In absence of operation of the pump 120, breathing gradually removes the substance from the material 92 by evaporation, so that eventually only air is breathed.

Referring to Figure 14, a breathing system that functions in accordance with the embodiment described in relation to Figure 13 is illustrated in an assembled form with shapes of parts modified.

In variant embodiments, the vaporisation unit 235 may include a plurality of the tubes 122 and the peristaltic pump 120 may be arranged to drive flow of the substance in the tubes 122. The tubes 122 are located to deposit the substance each at a different location on or in the material 192. The locations may be spaced transversely with the respect to a direction of flow and/or longitudinally.

In alterative embodiments, another type of pump may be used in place of the peristaltic pump 120 to drive fluid flow in the one or more tubes 122. For example, a piezo electric pump may drive flow. By way of other example, a pump including a venturi nozzle for dispensing fluid may be provided. Embodiments are not limited to any particular type of pump.

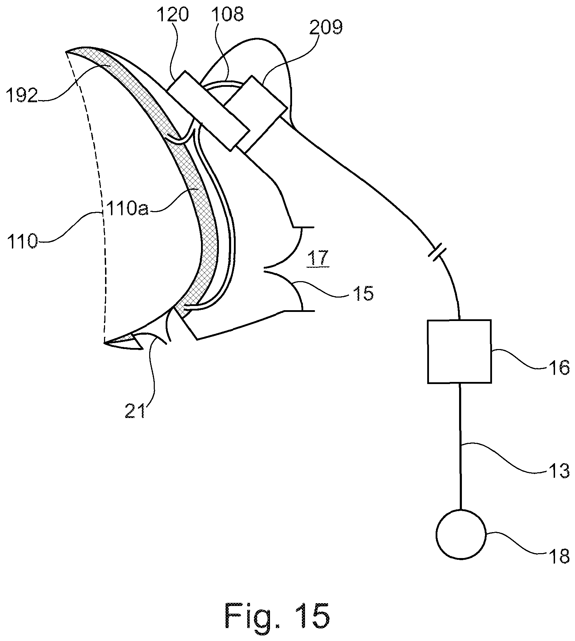

Referring to Figure 15, a breathing system is illustrated that functions in accordance with embodiments described above in relation to Figures 13 and 14, but in which there are a plurality of tubes 122. In this embodiment, the housing of the vaporisation unit 235 is integrally formed with the mask 110. The mask 110 may include a grating 110a over the opening 11 on which the material 192 can be mounted, for example with an adhesive at edges thereof. In an alternative, where the material 192 has sufficient structural rigidity, the material 192 may be located across the opening 11 without need for the grating 110a.

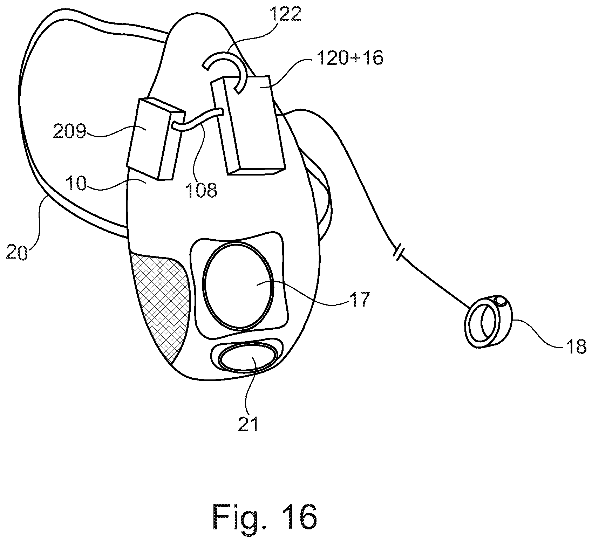

Referring to Figure 16, a breathing system that functions in accordance with the embodiment described in relation to Figure 15 is illustrated in an assembled form with shapes of parts modified.

The embodiments, variants thereof and modifications thereto described above disclose different ways in which the substance may be added to air (or another gas) through evaporation. Embodiments of the invention are not limited to any particular way in which the substance is added to air (or other gas) passing through the breathing system.

The substance may be a volatile liquid agent. Alternatively, the substance may be in the form of an inhalational powder where the vaporisation unit includes means operable to provide a controllable amount of the inhalation powder into the passing gas. For example, the inhalation powder may be added to passing gas as an aerosolised mist generated from a compressed gas or mechatronic mechanism.

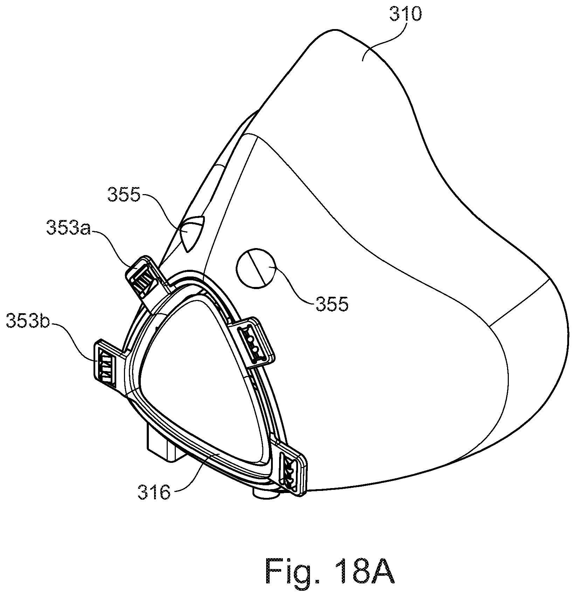

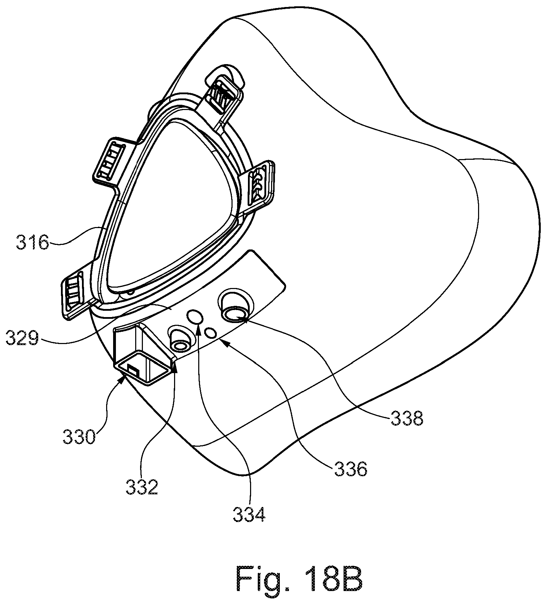

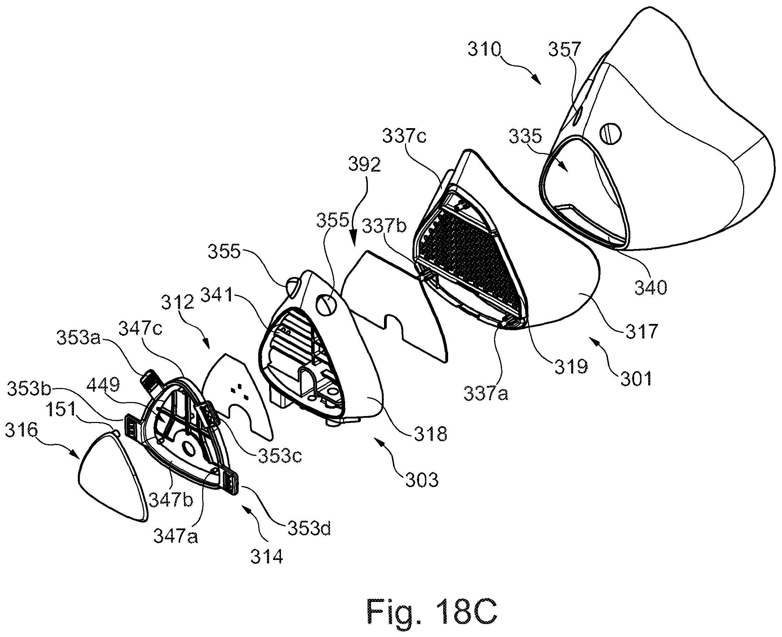

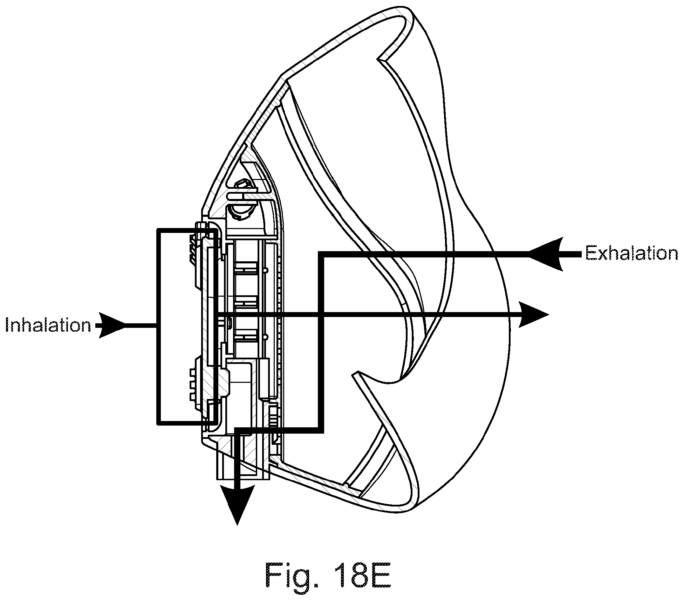

An example design of a breathing system in accordance with embodiments is now described with reference to Figures 18A to 18E. This example design may implement many of the embodiments described above or be easily modified to. The breathing system comprises a PAI in the form of a mask 310, a first housing part 301, a material 392 being part of the adding unit, a second housing part 303, an inspiratory valve 312, a third housing part 314 and a cover 316.

The mask 310 is, for example, formed of silicone, and is shaped to sealingly mount against a face of a patient over a mouth and nose. The mask 310 has an opening 335 at an end thereof remote from the mouth. The first and second housing parts 301, 303 have outer surfaces 317, 318 shaped to fit against an interior surface of the mask 310 and are located within the mask 310.

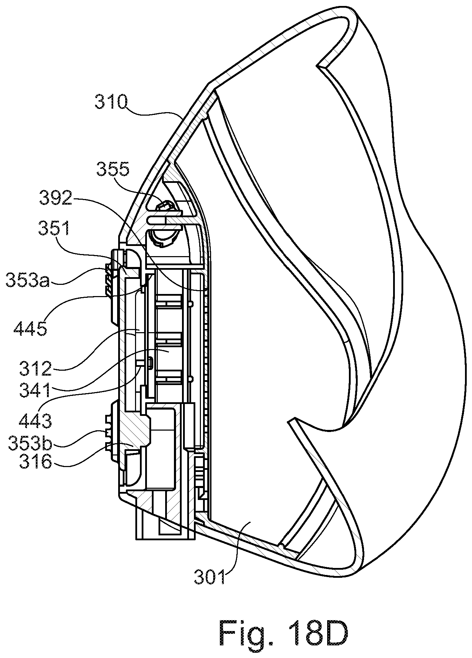

The first housing part 301 includes a first grating 319 that, when the first housing part 301 is fitted within the mask 310, is located spaced from and proximal to the mouth. The material 392 (which is functionally the material 192 or any variant thereof), is formed of cotton, for example, is planar, and fits against the first grating 319 on a side thereof remote from the mouth. The second housing part 303 and the first housing part 301 attach together. For this to occur, the first housing part 301 includes projections 337a-c arranged to engage in corresponding recesses (one being visible in Figure 18D and the others not being visible) in the second housing part 303. The first and second housing parts 301, 303 provide a space between them in which the material 392 is located. The second housing part 303 includes a second grating 341 for air to pass through.

The third housing part 314 locates at the opening 335 of the mask 310. The third housing part 312 includes a third grating 449 for air to pass through. The third housing part 312 also includes a wall 445 projecting in use towards a patient's mouth. The wall 445 is shaped to receive the inspiratory valve 312. The third housing part 314 includes three projections 443 that penetrate holes in the inspiratory valve 312 to fix it in place.

The cover 316 is attached to the third housing part 314 to prevent unintended ingress of objects, dirt, flies, etc. The cover 316 includes cylindrical projecting portions 151 configured to receive projections 347a-c extending from the third housing part 303. This results in the cover 316 being spaced from the third housing part 314, enabling inflow of air.

The third housing part 314 includes attachment portions 353a-d that extend outside of the mask 310. Straps can be attached to the attachment portions 353a-d to attach around the head of a patient.

As best seen in Figure 18D, the second housing part 303 has a projecting portion 329 including an exhalation port 330, an end tidal CO2 monitoring port 332, a light port 334, a substance adding port 336, and an oxygen port 338. The mask 310 has a hole 340 in which the projecting portion 329 is located, so that the ports 330, 332, 334, 336, 338 are each accessible to an attendant/operator. The exhalation port 330 enables outflow of exhaled gas. Although not shown in the Figures a carbon dioxide level sensor to enable end tidal carbon dioxide monitoring via the port 332. The oxygen port 338 enables connection of an oxygen supply, so oxygen can be added to air to be inhaled.

The control unit (not shown) and parts of the adding unit required to add the substance to the material 392 may be located outside of the mask 310 in embodiments and are not necessarily fixedly disposed relative to the PAI. They may be located within a housing for the user control 18, for example. The parts of the adding unit may be connected to the substance adding port 338, enabling the substance to be controllably added to the material 392. In alternative embodiments, such parts of the adding unit may be included in second housing part 303 (the mask can easily be adapted accordingly). In a variant, the reservoir may be located externally, for example co-located with the user control 18, and a pump internally, with the reservoir being connected to the substance adding port 338 and the pump being located in the second housing part 303 and configured to draw substance in and dispense the substance on the material 392. Additionally or alternatively, the control unit 16 may be located in the second housing part 303.

Although not shown, the control unit 16, whether located within the mask or externally, is operatively coupled to the user control 18, 118 described above. Input may be provided to the control unit by the user using the user control 18, 118 using any of the methods described above together with the breathing system of Figures 18A-E.

The first housing part 301, the material 392, the second housing part 303, the inspiratory valve 312 and the third housing part 314 all fit together and against the interior surface of the mask 310 to provide an inhalation path for inhaled air that must pass through the material 392. These parts are, where appropriate, sealed with respect to each other and the mask 310. The inhalation path is through the first, second and third gratings. An exhalation path passes through the exhalation port 330, to a filter (not shown) to prevent substance from being passed in surrounding air and disseminated.

To assembly the breathing system, the inspiratory valve 312 is attached to the third housing part 314, and the part 314 is located at the opening of the mask 310. The second housing part 303 with the material 392 is located within the mask 310.

The first housing part 301, the material 392, the second housing part 303 are located in the mask 310 against the part 314. The cover is then put in place.

Consciousness may be assessed in a manner mandating acknowledgement from a patient in the form of a user control indication. If acknowledgement is not provided, the controller may infer consciousness is impaired and act appropriately, for example, by decreasing the amount of drug given. Features now described enable this, but may be omitted in other embodiments.

The second housing part 303 includes two light emitters 355, e.g. LEDs. The mask 310 has corresponding apertures 357 into which each of the light emitters 355 locates. Alternatively, the mask 310 may include see-through material in place of these apertures, shaped so that the emitters 355 are visible at the exterior of the mask 310. The emitters 355 are positioned so that they can be easily seen by the patient using of the breathing system. These light emitters 355 are located so as to each be proximate a respective nostril of the patient.

The light emitters 355 are operatively connected to the control unit 16. Where the control unit is external to the mask, the lights are wired to the light port 334 and from there connected to the control unit.

The control unit 16 is configured to control adding of the substance by the adding unit also in dependence on whether the patient can follow instructions to operate the user control dependent on the status of the light emitters 355, which provides an indication of consciousness. The control unit 16 is configured to make a predetermined change to the status (e.g. by changing a characteristic) of the light emitters 355, for example turn them on or change their colour.

Embodiments are not limited to any particular method in which consciousness is monitored. By way of example, to check consciousness, the control unit 16 may be configured to monitor for consciousness and reduce the adding rate if consciousness is too low. This may be achieved by setting a timer and turning the light emitters on 355, and monitoring for a response from the user in accordance with instructions. The response may derive from a user input at the user control or by user of a separate user control. If a response is received, the control means may turn off the light. If a response is not received within a predetermined period, the control unit may determine that the consciousness level is low. The control means may then control the adding of a substance to the patient; the control unit 16 adjusts an amount of substance administered downwardly if the patient is unconscious or has a low consciousness level, and/or may adjust the amount upwardly if the patient has a high degree of consciousness. The monitoring requires such steps to be performed repeatedly.

In some embodiments, the control means is further configured to: measure time between making the predetermined changes to the emitted light and receiving a response. In this case, the determining of a consciousness level may be dependent on the measured time.

While the light emitters 355 are mounted in the mask 310, the light emitters 355 may be used with other types of PAI in other embodiments, provided the light emitters 355 are visible by the patient.

In alternative embodiments, instead of light emitters 355, other means for providing a stimulus to the patient may be used. In such an alternative embodiment, auditory or haptic feedback may be used. Where the stimulus is auditory, the breathing system includes a speaker system coupled to the control unit 16. Where the stimulus is haptic, the breathing system include a haptic feedback system coupled to the control unit 16.

In variant embodiments, it is not essential for the means providing the stimulus to be attached to a mask 310 or other PAI. Such means may be physically spaced from the PAI and connected to the control means 16 wirelessly or otherwise. Automated control of administration of a substance dependent on a determined consciousness is not limited to embodiments described herein and may find broader application.

As mentioned above, where the input to the control unit 16 is provided electronically, the user control 18 is attached to the control unit 16 with the cable 13. In variant embodiments, the user control 18 may alternatively be wirelessly coupled to the control unit 18. In this case, the user control 18 and the control unit 16 each includes transceiver circuitry enabling the signals to be sent from the user control 18 to the control unit 16. The signals may be sent using Bluetooth^{®}, ANT or any alternative suitable communications technology. Preferably the communications technology is a secure technology to prevent the possibility of third party interference in administration of doses.

As mentioned above, the user control 18 may be finger mounted. In variant embodiments, the user control 18 may be located in or affixed to a bracelet, necklace, or a device mountable on a finger tip. Alternatively, the user control 18 may be mountable on a tooth or teeth for bite operation by the patient. The user control may include a switch integrated into an adhesive patch, which is adherable to the patient's skin and can be operated by the patient by depressing or otherwise deforming. For example, pressure on the patch or movement of underlying skin may operate the switch. Like the user control 18 of other variant embodiments, the patch may be coupled to the control unit 16 in a wired or wireless manner.

The trigger signal sent from the user control 18 to the control unit 16 may be only indicative of the user control 18 being operated. In the above described embodiments the trigger signal is processed by the control unit 16 as indicating that more of the substance is to be administered. In variant embodiments, the trigger signal may include more information; for example the trigger signal may be indicative of the length of time for which a pressure sensitive switch in the user control 18 is depressed or a pressure valve from a range of possible values dependent on the pressure of depression. The user control 18 may in variant embodiments includes two or more pressure sensitive switches. In this case, the switches are configured to send respectively different signals to the control unit 18, indicating different operations are to occur, for example one indicating that a lesser amount of the substance is wanted and the other a greater.

In some embodiments, the user control 18 may be sufficiently large and bulky that it cannot be easily misplaced. Such a user control 18 may, for example, be useful for a patient with reduced consciousness or maturity, such as an infant. In this case, the switch may be incorporated within, for example, a squeezable toy with a pressure sensitive switch being located for operation in a squeezing action on the toy.

In other embodiments, the user control 18 may be operable other than with a pressure sensitive switch to send a trigger signal to the control unit 16. In an embodiment, the user control 18 may include audio detection unit such as a microphone. The audio detection unit is preferably mounted, directly or indirectly, on the mask 10, and carried by the mask 10. For example, the audio detection unit may be mounted on the vaporisation unit 12. The control unit 16 is in this case configured to respond to a signal from the audio detection unit, said signal being sent in response to detection of a predetermined audio signal from the patient. The predetermined audio signal may correspond to sounds of pain or distress from the patient, or from interpreted speech. Such an audio detection unit may also be used for patient communication where suitable transmission circuitry is also present, which may be useful in noisy environments, and/or to record words spoken by the patient and noises made by the patient. The audio detection unit may also be used to detect predetermined sounds indicative of malfunction of the apparatus, particularly leaks of gas from within the apparatus to the outside.

In another embodiment in which the user control 18 is not a pressure sensitive switch, the user control 18 is instead in the form of a digital camera. In use the digital camera is directed at the face of the patient. In this case, the control unit 18 is configured to determine one or more predetermined facial movements that the patient can perform, for example eye closure. Such a facial movement, or a sequence of such facial movements over maximum time period, such as a two blinks, may be used as a signal that release of more of the substance is wanted. Advantageously, this requires consciousness to perform.

The breathing system in accordance with any of the embodiments described above can, in variants thereof, include one or more sensor devices for monitoring one or more patient conditions such as vital signs. The one or more sensor devices may be configured to monitor parameters such as heart rate, blood pressure, oxygen saturation level, respiratory rate and/or patient consciousness level and to output a signal indicative of a value for the monitored parameter. The control unit 16 is configured to control the adding unit 14 dependent on the one or more parameters. In general, the algorithms may be configured to determine if further administration of the substance to the patient in view of the one or more parameters may have adverse effect on the patient, and, if so, not add the substance to the air in the airway 12 or reduce the amount. In a further embodiment, a sensor configured to detect the concentration of drug agent in inhaled gas may output a signal to the control unit. This signal may be additionally used to further optimise the administered dosage. For example, as the drug reservoir decreases, increased adding unit operation may be required to compensate.

In an embodiment a sensor device for monitoring patient consciousness level may be an EEG (electroencephalogram) sensor device, configured to monitor brain activity. The control unit 16 is configured to determine a level for consciousness based on signals received from the EEG sensor device.

The breathing system may include a digital camera connected to the control unit 16. The control unit 16 may be configured to use facial plethysmography to determine pulse, for example. One or more other sensors, such as a pulse oximeter, may be connected to the control unit 16 to provide the control unit 16 with indications of pulse rate and/or oxygen saturation level. The algorithms may be configured to determine amount of the substance to administer to the patient based on such indications.

In some embodiments, the breathing system includes a temperature sensor in the vaporisation unit, to monitor temperature therein. The temperature sensor is coupled to the control unit 16 to provide real-time temperature values to the control unit 16. In this case, the algorithms of the control unit 16 are configured to control the adding unit 14 dependent also on the temperature. This is because temperature affects rate of evaporation of the substance and/or because evaporation of the substance may have a cooling effect.

The breathing system may also comprise a user interface (other than the user control 18) operatively coupled to the control unit 16. The user interface may include one or more operator controls enabling input of information by the operator and/or means for communicating information to the patient and/or the operator. The one or more operator controls may be in the form of dials or a keyboard, for example. One of the operator controls may include an on/off switch for the breathing system.

An operator control may enable the operator to input a weight (or estimated weight) of the patient, for example the operator control may be in the form of a dial enabling this. The control unit 16 may be configured to determine the background rate dependent on the input weight. The operator control may alternatively enable input of information indicative of the background rate directly.

Additionally or alternatively, the operator control may enable the operator to input a height (or estimated height) of the patient. Additionally or alternatively, where the substance is to be administered for pain relief, the operator control may enable the operator to input information indicative of a pain level that the patient appears to the operator to be experiencing. The control unit 14 may be configured to use such weight, height and/or pain level information in determining a background rate and/or a rate of adding subsequent to operation of the user control 18 by the user.

The means for communicating information to the operator and/or to the patient may include a display and the control unit 16 can be configured to display information on the display. Additionally or alternatively, such means may include one or more lights and/or a speaker, the control unit 16 being configured to control the one or more lights and/or the speaker. The control unit 16 may control the display, the one or more lights and/or the one or more speaker to alert to the operator and/or to the patient with regard to patient safety and/or comfort.

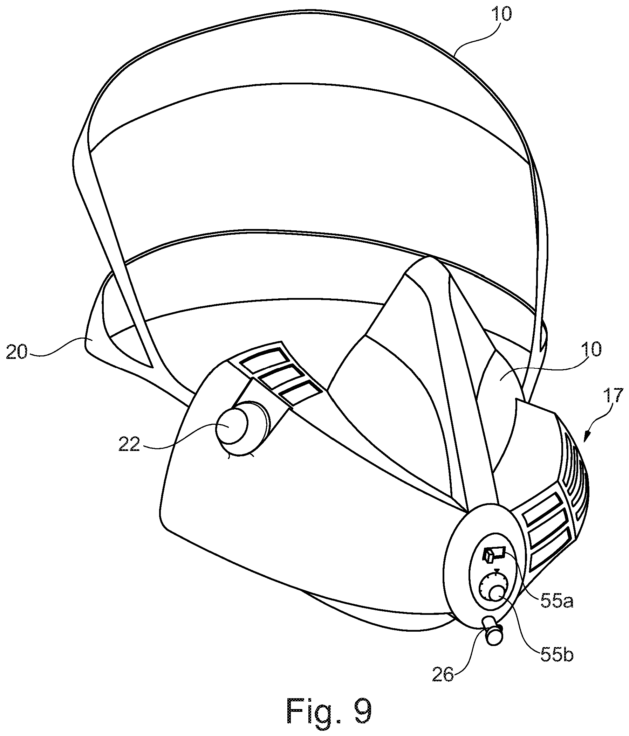

Referring to Figure 9, a breathing system that functions in accordance with the embodiment of Figure 10 is illustrated in an assembled form. A first of the operator control 55a enables the breathing system to be switched on and off. A second operator control 55b enables weight (or estimated weight) of the patient to be input.

In some embodiments, at least two volatile liquid substances may be located for vaporisation in the vaporisation unit. In other embodiments, the breathing may include two or more vaporisation units each containing a respective substance for adding to gas to be inhaled, the embodiments described above being adaptable for this with two or more vaporisation units being connected to the airway 12 in series or in parallel, depending on the embodiment. For example, one of the vaporisation units may be controllable to add a substance in the form of an analgesic agent to passing gas and another may be controllable to add a substance to treat hypotension caused by traumatic shock. In such cases, the amount of each substance administered to the patient may be independently controlled by the control unit 16, in dependence on the trigger signals and predetermined algorithms. In other embodiments, the amount of each substance added may be dependent on each other. In this case, the patient need only operate a single user control to control administration of both agents.

In the flow controls 30, 130, 230 described above, the gates are moveable by actuator assemblies including extendable pistons. In variant embodiments, one or more gates may be otherwise actuated. Embodiments are limited to any particular design of actuator. For example, one or more gates may be configured to be movable through electromagnetic induction caused by the actuator. In this case, when an electric current is removed, the respective biasing means causes the gate to return to its initial position in which air flow through the vaporisation unit 33 is prevented.

In embodiments, the vaporisation unit may also include a heating element to facilitate evaporation. Such a vaporisation unit may be used in evaporation of methoxyflurane and in the evaporation of other drugs. The control unit 16 may be configured to control the heating element to control the amount of substance vaporised for example by controlling degree of heating and/or the duration of heating. The heating element may comprise a heating coil. Other methods of controlling amount of substance vaporised may be known to persons skilled in the art; some methods known in the field of vaping may be used. Where there is heating, the substance need not be as easily evaporated as those substances used with a passive vaporisation unit. Any substance that can be transformed to a vapor from a liquid, a powder or another solid may be heated, although it is to be noted that some anaesthetic agents are flammable and in relation to these provision of a heating element may be undesirable.

### Control Processes

The control unit 16 may be configured to respond to receiving of an electronic trigger signal by first determining that a maximum rate will not be exceeded and, if not, increasing an adding rate at which the substance is added to gas to be inhaled. The adding unit 14 is controlled by the control unit 16 through provision of control instructions in accordance with the adding rate. The control unit 16 may be configured to control the adding unit to gradually decrease the adding rate in absence of received trigger signal.

The control unit 16 is configured to perform in a mode of operation with algorithms defining control loops. The control unit 16 is configured with stored variables that are dynamically adjustable by operation of one or more of the control loops. Embodiments are not limited to any particular way in which the control unit 16 controls the adding by the adding unit 14 dependent on the trigger signals from the user control 18. As will be appreciated by the skilled person, there are many control architectures that might usefully implement aspects of the embodiments.

The control unit 16 is configured with one of the variables in the form of an adding rate decrease value to cause the adding rate to gradually reduce to the background rate (or zero where no background rate is configured). The control unit 16 is configured to decrease the adding rate at time intervals, for example every 0.5 seconds, dependent on the adding rate decrease value. For example, the adding rate decrease value may be subtracted from the adding rate at said time intervals.

Another of the variables is in the form of an adding rate increase value. This value is the amount by which the adding rate is increased further to receipt of a trigger signal, subject to a predetermined maximum adding rate not being exceeded.

The adding rate increase value is adjustable so that, if the control unit 16 infers that the patient has a high degree of desire for the substance due to high frequency of received trigger signals, the adding rate increase value may be adjusted upwardly. In such circumstances, the control unit 16 may also decrease the adding rate decrease value so that the adding rate decreases more slowly.

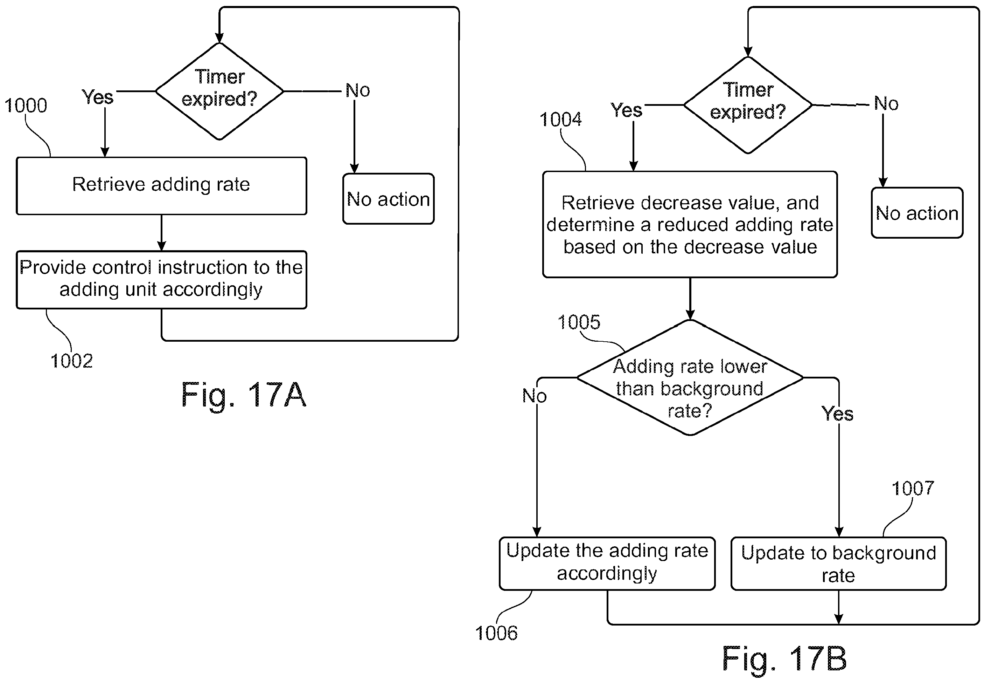

An example of how the control unit 16 may operate is now described with reference to Figures 17A-E for a control unit 16 of any embodiment in which the control unit 16 is electronic.

First, the control unit 16 determines a background rate. This may be further to input of information indicative of the background rate by operation of an operator control, for example the patient's weight. Alternatively, the background rate may be preconfigured and unchangeable.

The control unit 16 is configured to dynamically determine the adding rate and send control instructions. Embodiments are not limited to the adding rate being determined at any particular time intervals. The control loops may be performed at any predefined time interval that is small relative to breathing rate and may be performed at regularity of the clock rate of the processor of the control unit 16.

The patient is informed before commencing use of the user control 18 that, in the event of pain, the patient should provide an input to the user control 16, for example by pressing a button. Referring to Figure 17A, in a control loop the control unit 16 periodically, at predefined intervals, for example every 0.5 seconds, retrieves the adding rate at step 1000. The control unit 16 then provides a control instruction to the adding unit 14 to add the substance at the adding rate at step 1002.

Referring to Figure 17B, in a control loop the control unit 16 periodically, at predefined intervals, for example every 0.5 seconds, retrieves the adding rate decrease value at step 1004 and calculates a reduced adding rate based on the adding rate decrease value. For example, the control unit 16 may reduce the adding rate by the adding rate decrease value to calculate the reduced adding rate. The control unit 16 then determines at step 1005 if the calculated reduced adding rate is lower than the background rate. If not, the control unit 16 updates the adding rate with the determined reduced adding rate value at step 1006. Otherwise the control unit 16 updates the adding rate with the background rate at step 1007 (or makes no update if the adding rate is already the background rate).

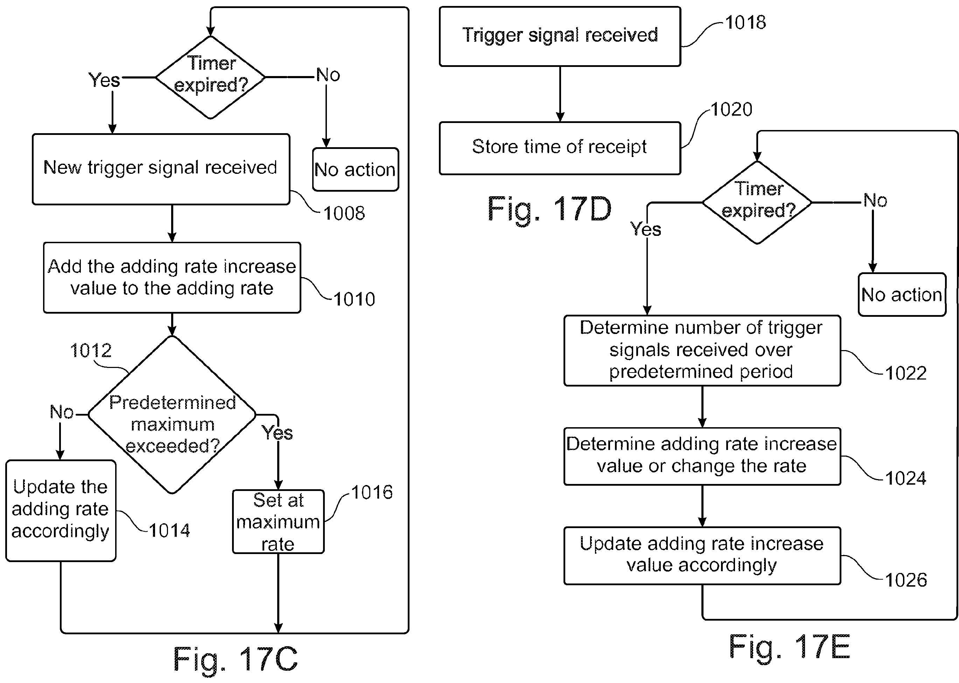

Referring to Figure 17C, in a control loop the control unit 16 periodically, at predefined intervals, for example every 0.5 seconds, updates the adding rate value dependent on whether a trigger signal has been received. At step 1008, the control unit 16 determines that a new trigger signal has been received. The control unit 16 then increases the adding rate by the adding rate increase value at step 1010. The control unit 16 then determines if a predetermined maximum adding rate is exceeded at step 1012. If so, the control unit 16 updates the adding rate value to the maximum adding rate at step 1016. Otherwise the control unit 16 updates the adding rate value to the increased adding rate at step 114.

In variant embodiments, the maximum adding rate may also be a variable, which the control unit 16 is configured to update in a control loop based on: input received from the operator via a coupled operator control; a monitored condition of the patient, for example the patient consciousness level, pulse rate, breathing rate, etc. For example, the maximum adding rate may be increased if the control unit 16 determines, based on the monitored conditions that no adverse effect is occurring to the patient and/or that the patient remains conscious.

Referring to Figure 17D, a trigger signal is received at step 1018. A time of receipt of the trigger signal is stored at step 1020.

Referring to Figure 17E, in a control loop the control unit 16 updates the adding rate increase value based on inference of the want of the patient for the substance. At step 1022, the control unit 16 determines the number of trigger signals received over a predetermined prior period. The predetermined period may be the previous 10 seconds, for example. The control unit 16 then determines an adding rate increase value dependent on the number of trigger signals at step 1024. For example, if the number of trigger signals is one, the control unit 16 updates or maintains the adding rate increase value at a first adding rate value. If the number trigger signals is greater than one, the control unit 16 updates the adding rate increase value to a second adding rate value that is greater than the first adding rate increase value. If there are no trigger signals, the control unit 16 determines to maintain or update the adding rate increase value at a third adding rate increase value lower than the first and second values.

In a variant control loop of Figure 17E, the number of times over predefined time periods that the patient is intended to operate the user control 16 is defined or is one of the variables. Where the number is a variable, the number may be settable by an operator via a user interface. Rather than the control unit 18 determining number of trigger signals over a predefined previous period, the control unit 18 determines the number over predefined time periods. The number is referred to as a "patent interaction frequency number" (PIFN). The PIFN may be set at three per minute, for example. If the number of inputs exceeds the PIFN, the adding rate increase value is increased and/or the adding rate decrease value decreased. If the number of inputs is less than the PIFN, the adding rate decrease value is increased and/or the adding rate increase value decreased. The result is that the control unit 16 is configured to change the adding rate such that the number of inputs by the patient moves towards the PIFN. In some embodiments, no background rate is required and steps relating to background rate may be omitted.

In other embodiments where a PIFN is set, the means for providing stimulus described above may also be included and control processes modified. In such embodiments, the patient is instructed, not only to provide an input to the user control 18 when experiencing pain, but what the stimulus is and to respond to it by also providing an input to the user control 18. This is to prevent administration of the substance to the patient such that the patient becomes drowsy or unconscious.

In use, first, the control unit 16 operates until the patient regularly provides inputs at a rate corresponding to the PIFN. Accordingly, the control unit 16 monitors if the rate of inputs corresponds over a predetermined period, for example three minutes where the PIFN is determined per minute. Where the rate of inputs is constant, an average adding rate represents a level at which the patient is comfortable. The control unit 16 thus determines the average rate. If the control unit 16 determines that the rate and the PIFN corresponds, the control unit 16 determines to change mode of operation. For the subsequent, other mode of operation, the control unit 16 ceases modifying the value for the adding rate using the adding rate increase and decrease values. Alternatively, for safety, the control unit 16 may be configured to modify the average value so it is slightly lower than the average value. Embodiments are not limited to the control unit 16 determining the average rate. An adding rate for use in the other mode may be otherwise determined based on the instantaneous adding rate and/or the adding rate over a previous period.

The control unit 16 then provides the stimulus to the patient at regular time intervals and monitors for an input from the patient in response. The control unit 16 also continues to control the adding unit to add the substance at the determined average rate. The monitoring is performed by setting of a timer and provision of the stimulus. If the timer expires before an input is received, the adding rate is reduced to zero or a significantly lower rate. This is because it is assumed that the patient has a low level of consciousness or is unconscious. In this case the control unit may also return to the original mode of operation. Optionally an alert/alarm is triggered. If a response is received before the timer expires, the process continues and the stimulus is provided at the next of the time intervals.

The stimulus may be in the form of turning on the light emitters described in relation to Figures 18A-E, or the other forms of stimulus mentioned above, and turning them off when a response is received. Embodiments are not limited to use of any particular form of stimulus.

In this other mode, the control unit 16 also determines if inputs are received other than in response to the stimulus, for example after a stimulus has been responded to but before a new stimulus is provided. If this occurs, the control unit 16 may return to the original mode of operation, since it is indicative that the adding rate is too low. In such an instance the need for the substance by the patient may have changed.

In a variant embodiment, if the timer expires without an input from the patient, the control unit 16 then causes provision of a stronger form of stimulus. For example, where the stimulus is a light emitter, the brightness or colour may change. Where the stimulus is auditory, volume may be increased and/or language (where language is output) may be made stronger. Where the stimulus is haptic, a level of vibration may increase. In some embodiments, more than one of the forms of stimulus may be provided as part of the breathing system and an additional or alternative form may be used to provide the stimulus for additional strength. With the provision of the stronger form of stimulus, another timer is started. Optionally, the adding rate is also decreased, for example by a predetermined proportion such as 50%.

The control unit 16 may be configured to monitor for regular need for use of the stronger form of stimulus in order to obtain a response from the user. In this case, the control unit 16 may reduce the adding rate.

If an input is not received from the user before the other timer expires, the adding rate is reduced to zero or a significantly lower rate. Optionally an alert/alarm is triggered. The control unit 16 may also return to the original mode of operation. If an input is received, the adding rate may be maintained.

Such embodiments need not be implemented in a breathing system where a substance is added to a gas. Such embodiments may be implemented in other systems where a substance is to be administered to a patient. In such systems the substance may be administered intravenously, for example. The amount of substance administered may be controlled dependent at least on responses (or non-responses) to stimuli. Such substances may have sedative, anaesthetic and/or analgesic effect on the patient.

The same control loop of Figure 17E may also run where the predetermined time period is longer and may be from the start of operation of the breathing system. Alternatively, the control loop of Figure 17E may also run in multiple configurations concurrently. For example, in one configuration, the predetermined time period is longer and in the other configuration the time period is shorter. The outputs of each control loop configuration may be differentially weighted. For example, the long time period configuration may be less heavily weighted in comparison to the output from the shorter time period configuration. The control unit 16 integrates the weighted outputs from both control loops to operate the adding unit. In this way, long term use of the breathing system establishes a baseline set of variables. The variables may be perturbed in the short term, for example, in the instance of sudden significant desire for the substance. However, the variables rapidly revert back to the established baseline when the sudden short term want has passed and drug administration desire goes back to baseline.

In some embodiments of the invention described above, operation of the adding unit may be controllable in a binary manner. In such cases, alternative control loops may be used, as would be apparent by the skilled person. Alternatively, rate may be controlled by repeated switching between the one and other configurations.

### Alerts

As mentioned above, the control unit 16 may alert the operator and/or the patient with regard to patient safety and/or comfort and the algorithms include an alert function in this respect. If using the alert function the control unit 16 determines that an alert condition is met, the control unit 16 causes the alert to be communicated to the operator and/or to the patient via the user interface.

The control unit 16 may be configured to determine if the number of trigger signals received over a predetermined preceding period is above a threshold number. If so the control unit 16 is configured to determine the alert condition is met. Where the substance is an analgesic agent, this may be indicative of insufficient analgesia. The control unit 16 may perform this determining in response to receipt of a new trigger signal.

The control unit 16 may be configured to determine if the adding rate is at the maximum rate, and if so to determine that an alert condition is met.

The control unit 16 may be configured to check whether any trigger signals have been received within a predetermined time period, for example ten minutes. If none is received, the control unit 16 is configured to determine that an alert condition is met. This may be indicative of a low level of consciousness.

Additionally, the control unit 16 may be configured to monitor the amount of substance that has been added since the start of operation of the breathing system. If the amount exceeds a maximum amount, which may be dependent on the length of operation, the control unit may determine that an alert condition is met.

Alerts may function independently of the control of the adding unit. For example, in a simple switch operated binary adding unit, the adding unit may be operated without restriction according to switch manipulation. A controller may recorded the timing and pattern of user control inputs and provide the appropriate alarms without effect on the adding unit. In this example, in the event of an alarm, an attending clinician is required to assess the issue and act appropriately.

In the above described control loops, it is assumed that the trigger signals are discrete and are a simple "want" signal. In variant embodiments, the trigger signal may be continuously sent for a time period for which a switch is operated (e.g. a pressure switch depressed) and the length of the time period may be indicative of the amount of the substance wanted. The control loops may be modified to take this into consideration, as will be clear to the skilled person. The term "input" herein should be construed to encompass such trigger signals. It is also assumed that the trigger signals indicate only that further substance is wanted by the patient and not an amount of the substance. In variant embodiments, the user control 18 may be configured to indicate an amount, for example dependent on the pressure exerted on a pressure switch where high pressure indicates that a higher amount is wanted. As another example, the user control may include a plurality of switches and selection of a particular one may correspond with wanting higher or lower amounts. Again, the control loops may be modified to take this into consideration, as will be clear to the skilled person.

In embodiments where the temperature sensor is provided, the adding rate may also be dependent on the determined temperature.

In a variant embodiment, the adding unit may include the airflow control 30, 130, 230 described above and, in place of a vaporisation unit, a gas source is provided for provision of a gas to the airflow control for mixing with air. The gas has sedative, anaesthetic and/or analgesic effect on the patient. The gas may be nitrous oxide.

Manufacturing processes for manufacture of the breathing systems in accordance with the above described embodiments will be apparent to persons skilled in the art. For example, the masks may be formed of a hard plastics material or silicone, and the airways of hard plastics material. Embodiments are not limited to use of specific materials.

Various modifications to the embodiments described above will be apparent to the skilled person.

When used in this specification and claims, the terms "comprises" and "comprising" and variations thereof mean that the specified features, steps or integers are included. The terms are not to be interpreted to exclude the presence of other features, steps or components.

Unless otherwise stated, all individual features and/or steps of all embodiments described herein are disclosed in isolation and any combination of two or more such features is also disclosed, to the extent that such features or steps or combinations of features and/or steps are capable of being carried out based on the present specification as a whole in the light of the common general knowledge of a person skilled in the art, irrespective of whether such features or steps or combinations of features and/or steps solve any problems disclosed herein.

Embodiments of the invention may be defined in the following clauses:
1. A breathing system for a patient to inhale an analgesic, anaesthetic and/or sedative substance from, comprising:
   a patient airway interface mountable on the patient so that inhaling and exhaling occur through the patient airway interface;
   adding means for adding the substance to gas to be inhaled, wherein the substance is provided in liquid or powder form, comprising vaporisation means for vaporising of the substance into the gas;
   a user control operable by the patient;
   a control means, the user control being coupled to the control means to provide inputs thereto in response to operation of the user control by the patient;
   wherein the control means is configured to store information indicative of a time of received inputs and/or of amounts of the substance added, and to control the adding by the adding means dependent at least on:
      a most recently received input received from the user control, and
      the stored information indicative of the times of previously received inputs and/or of the amounts of the substance added further to the previously received inputs.
2. The breathing system of clause 1, wherein the control means is configured to:
   determine, dependent on said stored information and the most recently received input, to increase or decrease the amount of the substance added to the gas; and
   control the adding unit accordingly.
3. The breathing system of clause 2, wherein the control means is configured to increase the amount of the substance added to the gas if a frequency of the inputs over a predetermined first time period is greater than a first threshold, and/or to decrease the amount of the substance added to the gas if the frequency of the inputs over a predetermined second time period is less than a second threshold.
4. The breathing system of any one of the preceding clauses, wherein the control means is coupled to an alerting means, wherein the control means is configured to determine an alert condition, and to cause communication of the alert condition to the patient and/or an operator dependent on at least one of the following rules being met:
   - an input from the user control is not received within a predetermined period;
   - a rate of inputs received at the control means exceeds a threshold rate;
   - the control means determining that the substance is to be added at a rate exceeding a predetermined maximum rate;
   - a rate of inputs received at the control means exhibits a sudden change in pattern;
   - a predetermined maximum amount of the substance has been added to the gas.
5. The breathing system of any one of the preceding clauses, wherein the vaporisation means is arranged to spread the substance, to increase surface area thereof, thereby to facilitate evaporation of the substance.
6. The breathing system of clause 5, wherein the vaporisation means comprises a material located for gas to flow over or through it, and from which the substance can evaporate, wherein the vaporisation means is configured to disperse the substance on and/or through the material.
7. The breathing system of clause 6, wherein the material is such as to wick the substance from a reservoir of the substance, thereby to disperse the substance through the material.
8. The breathing system of clause 6, wherein the vaporisation means further comprises a spraying device for spraying the substance onto the material.
9. The breathing system of clause 6 or 7, wherein the adding means comprises a pump means for pumping the substance onto or into the material.
10. The breathing system of clause 6, 7 or 9, wherein the pump means comprises a plurality of tubes opening at a plurality of spaced locations on or through the material, wherein the pump means is configured to pump the substance through the tubes to deposit the substance at said spaced locations.
11. The breathing system of any one of the preceding clauses, wherein the vaporisation means comprises a heater for heating the substance to cause or facilitate the vaporisation.
12. The breathing system of any one of clauses, further comprising an airway coupled to the patient airway interface including an opening for intake of gas, wherein inhalation causes intake of said gas into the airway, wherein the adding means is arranged to add the substance to the gas in the airway.
13. The breathing system of any one of the preceding clauses, wherein the adding means further comprises a flow control configured to prevent or permit flow of the gas with the substance in into the patient airway interface from the vaporisation means, and to permit or prevent flow of gas without the substance into the patient airway interface, wherein the control means is coupled to the flow control and is configured to control the flow control.
14. The breathing system of clause 13, wherein the flow control is configured with at least one gate, wherein the flow control including the at least one gate has a first configuration in which flow from the vaporisation chamber is prevented and flow from the airway is permitted, and a second configuration in which flow from the vaporisation chamber is permitted and flow from the airway is prevented, and wherein the flow control is controllable to move the flow control between the first and second configurations.
15. The breathing system of clause 12, wherein an intake of the vaporisation means is coupled to the airway, such that gas in the vaporisation chamber is drawn from the airway.
16. The breathing system of any one of the preceding clauses, further comprising stimulus provision means for providing stimulus to the patient, coupled to the control means, wherein the control means is configured to control the adding by the adding means also in dependence on inputs by the user in response to the stimuli..
17. The breathing system of one of clauses 15 to 16, wherein the flow control and/or the control means is configured to increase or decrease gas flow via the vaporisation means in inverse relation to increase or decrease of flow from the airway.
18. The breathing system of any one of the preceding clauses, wherein the patient airway interface is provided separately to at least some of the rest of the breathing system; wherein the patient airway interface includes a first connector and the rest of the breathing system includes a second connector connectable to the first connector to enable gas flow from the connector into the patient airway interface.
19. The breathing system of any one of the preceding clauses, wherein at least one of: the vaporisation means; adding means; and the control means are mounted on the patient airway interface, so as to be mountable on a face of the patient..
20. The breathing system of any one of the preceding clauses, wherein the control means is configured to control the adding unit to add the substance to gas at at least a background rate.
21. A method in a breathing system for a patient to inhale an analgesic, anaesthetic and/or sedative substance from, the breathing system comprising:
   a patient airway interface mountable on the patient so that inhaling and exhaling occur through the patient airway interface;
   adding means for adding the substance to gas to be inhaled, wherein the substance is provided in liquid or powder form, comprising vaporisation means for vaporising of the substance into the gas;
   a user control operable by the patient;
   a control means, the user control being coupled to the control means to provide inputs thereto in response to operation of the user control by the patient,
      wherein the control means is configured to store information indicative of a time of received inputs and/or of amounts of the substance added, wherein the method comprises:
      controlling the adding by the adding means dependent at least on:
      a most recently received input received from the user control, and
      the stored information indicative of the times of previously received inputs and/or of the amounts of the substance added further to the previously received inputs.
22. A computer program product controlling adding of an analgesic, anaesthetic and/or sedative substance to air in a breathing system, which, when comprising computer program code which, when executed by processing apparatus, performs:
   storing of information indicative of a time of received inputs from a user control operable by a patient, and/or of amounts of the substance added; and
   controlling the adding by the adding means dependent at least on:
      a most recently received input received from the user control, and
      the stored information indicative of the times of previously received inputs and/or of the amounts of the substance added further to the previously received inputs.
23. A breathing system for a patient to inhale an analgesic, anaesthetic and/or sedative substance from, comprising:
   a patient airway interface mountable on the patient so that inhaling and exhaling occur through the patient airway interface;
   adding means for adding the substance to gas to be inhaled, wherein the substance is provided in liquid or powder form, comprising vaporisation means for vaporising of the substance into the gas, wherein the vaporisation means is mounted in or on the patient airway interface so as to be mountable on a face of the patient;
   a user control operable by the patient;
   a control means, the user control being coupled to the control means to provide inputs thereto in response to operation of the user control by the patient, wherein the control means is configured to control adding by the adding means using the vaporisation means dependent at least on the inputs.
24. The breathing system of clause 23, wherein the vaporisation means comprises a material located for gas to flow over or through it, and from which the substance can evaporate, wherein the adding means is configured to locate the substance on and/or through the material.
25. The breathing system of clause 24, further comprising a housing mounted on or in the patient airway interface so as to be mountable on the face of the patient, wherein the housing retains the material and provides an inhalation path through or over the material.
26. The breathing system of clause 25, wherein the material is planar, wherein the breathing system is configured for mounting of the material proximal the face of the patient, and wherein the inhalation path passes perpendicularly with respect to the plane of the material.
27. The breathing system of any one of clause 23 to 26, wherein the adding means is mounted on or in the patient airway interface so as to be mountable on a face of the patient.
28. The breathing system of any one of clauses 23 to 27, wherein the vaporisation means includes a substance intake port and the adding means comprises pumping means for pumping the substance to the material through the substance intake port.
29. The breathing system of any one of clauses 23 to 28, wherein the pumping means is located physically spaced from the patient airway interface, optionally coupled to and co-located with the user control, and coupled to the substance intake port.
30. The breathing system of any one of clauses 23 to 29, wherein the control means is coupled to and at least partially co-located with the user control.
31. The breathing system of any one of clauses 23 to 29, wherein the control means is at least partially mounted on or in the patient airway interface so as to be mountable on a face of the patient.

## Claims

1. A breathing system for a patient to inhale an analgesic, anaesthetic and/or sedative substance from repeatedly and consecutively, comprising:
a mask mountable on the patient, so that inhaling and exhaling occur through the mask;
adding means for adding the substance to a gas to be inhaled, wherein the substance is a volatile liquid, comprising vaporisation means for vaporising of the substance into the gas, wherein the vaporisation means is mounted in or on the mask so as to be fixedly disposed relative thereto and mountable on a face of the patient,
a user control;
a control means, the user control being coupled to the control means to provide input thereto in response to operation of the user control by the patient, and wherein the control means is configured to control the adding by the adding means using the vaporisation means dependent at least on the input.

2. The breathing system of claim 1, further comprising the substance.

3. The breathing system of claim 1 or claim 2, wherein the vaporisation means comprises a material located for gas to flow over or through, and from which the substance can evaporate, wherein the adding means is configured to locate or disperse the substance on and/or through the material.

4. The breathing system of claim 3, further comprising a housing mounted on or in the mask so as to be mountable on the face of the patient, wherein the housing retains the material and provides an inhalation path through or over the material.

5. The breathing system of claim 4, wherein the material is planar, wherein the breathing system is configured for mounting of the material proximal the face of the patient, and wherein the inhalation path passes perpendicularly with respect to the plane of the material.

6. The breathing system of any one of the preceding claims, wherein the mask includes an inspiratory valve and an expiratory valve, wherein the inspiratory valve is a one-way valve located to prevent flow of exhaled air through the vaporisation means and to permit inhalation of gas through the vaporisation means, and wherein the expiratory valve is a one-way valve enabling exhaled gas to exit the patient mask to the environment and prevents ambient air from entering the breathing system.

7. The breathing system of any one of the preceding claims, wherein the vaporisation means includes a substance intake port and the adding means comprises pumping means for pumping the substance to the material through the substance intake port.

8. The breathing system of claim 7, wherein the pumping means is located physically spaced from the mask and coupled to the substance intake port.

9. The breathing system of claim 8, wherein the pumping means is coupled to and co-located with the user control.

10. The breathing system of any one of claims 1 to 7, wherein the adding means is mounted on or in the mask so as to be mountable on a face of the patient.

11. The breathing system of any one of claims 8 and 9, comprising a reservoir for containing the substance, wherein the pumping means is coupled to the reservoir for pumping the substance from the reservoir to the material.

12. The breathing system of any one of the preceding claims, wherein the control means is coupled to and at least partially co-located with the user control.

13. The breathing system of claim 12, wherein the user control is physically spaced from the mask and operatively coupled to the at least part of the control means mounted on or in the mask by a cable.

14. The breathing system of any one of the preceding claims, further comprising at least one sensor device for detecting a condition of the patient and providing sensor information indicative of the condition to the control means, wherein the control means is configured to control the adding means dependent also on the sensor information.

15. The breathing system of any one of claims, further comprising an airway coupled to the mask including an opening for intake of gas, wherein inhalation causes intake of said gas into the airway, wherein the adding means is arranged to add the substance to the gas in the airway.
